(19) Europäisches Patentamt
European Patent Office
Office européen des brevets

(11) **EP 4 512 893 A1**

(12) **EUROPEAN PATENT APPLICATION**

(43) Date of publication:
**26.02.2025 Bulletin 2025/09**

(21) Application number: **23192547.0**

(22) Date of filing: **22.08.2023**

(51) International Patent Classification (IPC):
***C12N 9/38*** *(2006.01)*

(52) Cooperative Patent Classification (CPC):
**C12N 9/2402; C12Y 302/0108;** C07K 2319/02; C07K 2319/20; C07K 2319/35; C07K 2319/50

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **AB Enzymes Finland Oy**
  **05200 Rajamäki (FI)**
- **OY Karl Fazer AB**
  **00941 Helsinki (FI)**

(72) Inventors:
- **MÄKINEN, Susanna**
  **05200 Rajamäki (FI)**
- **JUNTUNEN, Kari**
  **05200 Rajamäki (FI)**
- **SEEFRIED, Michael**
  **64293 Darmstadt (DE)**
- **LAINE, Jarmo**
  **00941 Helsinki (FI)**
- **LOPONEN, Jussi**
  **00941 Helsinki (FI)**
- **PURANEN, Terhi**
  **05200 Rajamäki (FI)**
- **ALAPURANEN, Marika**
  **05200 Rajamäki (FI)**

(74) Representative: **Espatent Oy**
**Kaivokatu 10 D**
**00100 Helsinki (FI)**

Remarks:
The complete document including Reference Table(s) and the Sequence Listing(s) can be downloaded from the EPO website

# (54) FRUCTANASE VARIANTS

(57) A variant polypeptide of fructanase, a fusion protein and an enzyme composition comprising said variant polypeptide, recombinant host cell producing the variant polypeptide, method for manufacturing the variant polypeptide, a use of the variant polypeptide to degrade and modify fructan containing material, and a premix for baking comprising the variant polypeptide.

**Fig. 1**

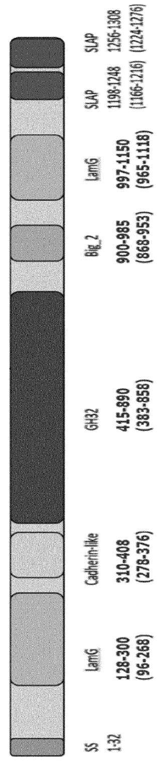


EP 4 512 893 A1

## Description

### TECHNICAL FIELD

The present disclosure generally relates to field of enzyme technology. The disclosure relates particularly, though not exclusively, to fructanase variants having an increased productivity and/or fructan degradation activity. The present fructanase variants are useful in various applications where degradation of fructan is desired, such as in plant-based and baked products.

### BACKGROUND

This section illustrates useful background information without admission of any technique described herein representative of the state of the art.

Fructans are comprised of fructose residues, normally with a terminal sucrose unit (i.e. a glucose-fructose disaccharide). The linkage position of the fructose residues determines the type of the fructan. The basic types of single-linkage fructans are inulin (fructosyl units linked by β-2,1-linkages) and levan (or phlein, both linked by β-2,6-linkages). Additionally, there exists a mixed-linkage fructan called graminan (containing both β-2,1-linkages and β-2,6-linkages). Common sources of fructans include plants, such as cereals, vegetables, fruits, and nuts.

Several fructan degrading enzymes, i.e., fructanases, are known in the art. Fructanases are fructosidases or exo-beta-D-fructosidases, which are enzymes that catalyze the hydrolysis of a fructan. More specifically, fructanase catalyzes the hydrolysis of terminal, non-reducing (2->1)- and (2->6)-linked beta-D-fructofuranose residues in fructans. Substrates of fructanase include fructans such as inulin and levan, and sucrose.

The use of fructanases for degradation of fructans in plant-based materials have been described for various purposes. Fructanases can be added to hydrolyze fructans, for example, to achieve increased softness of the food, or to bring additional sweetness to the food. In WO2017220864 fructanases are used to degrade fructans, to obtain foodstuff suitable for a diet low in fermentable carbohydrates, known as FODMAP.

However, fructanase enzymes are often produced in insufficient quantities, the production yields thereby not being economically feasible. Moreover, the activity of the known fructanase enzymes may be low and inadequate to be used, for example with foodstuff, to hydrolyze fructans efficiently. Therefore, new fructanase enzymes are needed, having higher production level and specific fructan degrading activity.

It is an object of the present disclosure to provide fructanase variants that exhibit fructanase activity and that have improved properties that allow their use in industrial processes. Yet another object of the present disclosure is to provide fructanase variants that can be used in enzyme compositions and in foodstuff for fructan degradation.

### SUMMARY

The appended claims define the scope of protection. Any examples and technical descriptions of apparatuses, products and/or methods in the description and/or drawings not covered by the claims are presented not as embodiments of the invention but as background art or examples useful for understanding the invention.

The present disclosure provides a variant polypeptide with fructanase activity, being able to efficiently degrade fructan. The disclosure relates particularly, though not exclusively, to fructanase variants having an increased activity and/or productivity.

According to a first aspect there is provided a variant polypeptide having:

fructanase activity;
an amino acid sequence having at least 80 % amino acid sequence identity with the amino acids 383 - 858 of SEQ ID NO: 1; and
at least one substitution, insertion or deletion of an amino acid, the at least one substitution, insertion or deletion preventing N-glycosylation of an amino acid of the variant polypeptide at an amino acid position corresponding to the amino acid position 672 of SEQ ID NO:1, when expressed in a host cell capable of N-glycosylation.

In an embodiment, the amino acid numbering of the amino acid having N-glycosylation prevented by the at least one substitution, insertion or deletion of an amino acid, corresponds to the amino acid numbering of the SEQ ID NO: 1. In an embodiment, the amino acid numbering of the at least one substitution, insertion or deletion of an amino acid of the variant polypeptide corresponds to the amino acid numbering of the SEQ ID NO: 1. In an embodiment, the amino acid numbering of the variant polypeptide corresponds to the amino acid numbering of the SEQ ID NO: 1.

It was surprisingly found that by preventing N-glycosylation at a specific amino acid position of the variant polypeptide via at least one substitution, insertion or deletion of an amino acid, the activity and/or productivity of the variant polypeptide in production hosts is significantly increased.

According to a second aspect there is provided a fusion protein comprising the variant polypeptide according to the first aspect and at least one:

an amino acid sequence providing a secretory signal sequence;
an amino acid sequence which facilitates purification, such as an affinity tag or His-tag;
an amino acid sequence controlling and enhancing the production of the fusion protein, such as a carrier polypeptide;
an amino acid sequence encoding a protease cleavage site;
a linker sequence;
an amino acid sequence having an enzyme activity; and/or
an amino acid sequence providing for the fusion protein with binding affinity, such as a carbohydrate binding moiety.

According to a third aspect there is provided an enzyme composition comprising the variant polypeptide of the first aspect or the fusion protein of the second aspect.

According to a fourth aspect there is provided a recombinant host cell comprising genetic elements that allow producing at least one variant polypeptide of the first aspect, or the fusion protein of the second aspect.

According to a fifth aspect there is provided a method of manufacturing the variant polypeptide of the first aspect, or the fusion protein of the second aspect, comprising: cultivating the recombinant host cell of the fourth aspect in conditions allowing production of the at least one variant polypeptide of the first aspect, or the fusion protein of the second aspect; and optionally recovering the variant polypeptide or the fusion protein.

According to a sixth aspect there is provided a use of the variant polypeptide of the first aspect, and/or the fusion protein of the second aspect and/or the enzyme composition of the third aspect, for degradation of fructan in plant-based material.

According to a seventh aspect there is provided a premix for baking, comprising the variant polypeptide of the first aspect, and/or the fusion protein of the second aspect and/or the enzyme composition of the third aspect.

Different non-binding example aspects and embodiments have been illustrated in the foregoing. The embodiments in the foregoing are used merely to explain selected aspects or steps that may be utilized in different implementations. Some embodiments may be presented only with reference to certain example aspects. It should be appreciated that corresponding embodiments may apply to other example aspects as well.

## BRIEF DESCRIPTION OF THE FIGURES

Some example embodiments will be described with reference to the accompanying figures, in which:

Fig. 1 schematically shows a picture of the *Lactobacillus crispatus* LFRU fructanase with multiple putative domains depicted, according to an example embodiment. The amino acid numbering in parenthesis indicates the amino acid numbering of different domains the wild type *Lactobacillus crispatus* LFRU fructanase without the signal sequence (SS) according to SEQ ID NO:1. The numbers without parentheses indicates the amino acid numbering of the different domains of the same LFRU fructanase including the signal sequence amino acids 1-32. The domains comprised by the wild type *Lactobacillus crispatus* LFRU fructanase include signal sequence, two LamG, one Cadherine-like, one GH32, one Big 2, and two SLAP domains.

Fig. 2 shows a schematic picture of the expression plasmid used in the transformation of *T. reesei* for expression of fructanase (wildtype and variants) genes (= GOI, Gene of interest). Expression of the recombinant fructanase genes in a host cell is controlled using the following genetic elements comprised by the plasmid: *T. reesei* cel7A promoter (Pcel7A) for transcription initiation, and *T. reesei* Cel6A (Tcel6A) terminator for transcription termination. *T. reesei* cel6A carrier encoding a Cel6A CBM and a linker region was used instead of the native fructanase signal sequence with a the Kex2 protease cleavage site (kex2) included between the encoded carrier polypeptide and the fructanase. The synthetic amdS marker gene encoding for acetamidase was included for selection of the transformants, and *T. reesei* cel7A 5'- and 3'-flanking regions were used for optional targeting of the expression construction to cel7A locus. The picture was generated using Geneious Prime 2021 created by Biomatters.

Fig. 3 shows an SDS-polyacryl amide gel prepared with samples of shake flask culture supernatants of *T. reesei* strains producing wildtype LFRU fructanase protein (lane 3) and fructanase variants LFG (lane 4), LFS (lane 5), LF2 (lane 6), LF3 (lane 7), LF4 (lane 8), LF5 variant (lane 9), and LF3GS (lane 10). Lane 2 contains a sample from an empty host strain without any fructanase production and lane 1 contains a standard SDS-PAGE molecular weight marker (Benchmark™ Protein Ladder, Thermo Fisher Scientific).

Fig. 4 shows a polypeptide sequence alignment of fructanases from genus *Lactobacillus.* Wildtype fructanase sequences were aligned to determine the position for N-glycosylation mutation. In *L. amylovorus* (SEQ ID NO: 9) and *L. crispatus* (SEQ ID NO: 12) amino acid sequences the amino acid position T655 and T631, respectively, correspond to L. *crispatus* LFRU amino acid at position T674 (SEQ ID NO: 1). The second *L. crispatus* fructanase (SEQ ID NO: 12) analyzed has an identity of 99 % to the reference fructanase LFRU (SEQ ID NO: 1).

Fig. 5 shows an SDS-polyacryl amide gel prepared with samples of shake flask culture supernatants of *T. reesei* strains producing fructanases from other *Lactobacillus* strains: *L. amylovorus* wildtype fructanase LFM24 (lane 3), the variant LFM24GS thereof with C-terminal truncation and N-glycosylation mutation (lane 4), and *L. crispatus* variant LFM33GS (lane 5). Lane 2 contains a sample from an empty host strain without any fructanase production and lane 1 contains a standard SDS-PAGE molecular weight marker (Benchmark™ Protein Ladder, ThermoFisher Scientific).

Fig. 6 In (A) is shown the pH dependency of fructanase activity of the indicated heterologously produced variant fructanases LF3, LFM24GS and LFM33GS. The pH dependency of the fructanase activity was determined from the culture supernatant using inulin as substrate in a 10 min reaction at 40 °C. The reaction pH is shown in the x-axis, whereas the relative fructanase activity is shown on the y-axis.

In (B) is shown the temperature dependency of fructanase activity of the same heterologously produced variant fructanases as in (A). Temperature optimum was determined at optimal pH of 5.5. The duration of the reaction was 10 min containing inulin as a substrate. The reaction temperature is shown in the x-axis, whereas the relative fructanase activity is shown on the y-axis.

In (C) is shown the substrate specificity of the same heterologously produced variant fructanases as in (A) and (B), measured as relative fructanase activity. The substrate specificity was determined by HPLC and by using separately both levan and inulin as substrates. Levanase activity was set as the 100 % reference for each measured variant, respectively. The fructanase variant molecules are indicated in the x-axis, whereas the relative fructanase activity is shown on the y-axis. The details of this analysis are described in Example 4.

**SEQUENCE LISTING**

SEQ ID NO: 1 Amino acid sequence of a full-length *Lactobacillus crispatus* fructosidase LFRU without a signal peptide, contains the amino acids 1-1279.

SEQ ID NO: 2 LFG, amino acid sequence of a full-length *Lactobacillus crispatus* fructosidase without a signal peptide, comprising a mutation T674A.

SEQ ID NO: 3 LFS, amino acid sequence of a truncated (SLAP domains removed) *Lactobacillus crispatus* fructosidase variant without signal peptide, contains the amino acids 1-1165 of the full-length wild type fructosidase.

SEQ ID NO: 4 LF2, amino acid sequence of a truncated (SLAP domains removed) *Lactobacillus crispatus* fructosidase of SEQ ID NO:3, comprising the mutation N672Q.

SEQ ID NO: 5 LF3, amino acid sequence of a truncated (SLAP domains removed) *Lactobacillus crispatus* fructosidase of SEQ ID NO:3, comprising the mutation T674A.

SEQ ID NO: 6 LF4, amino acid sequence of a truncated (SLAP domains removed) *Lactobacillus crispatus* fructosidase of SEQ ID NO:3, comprising the mutation T674G.

SEQ ID NO: 7 LF5, amino acid sequence of a truncated (SLAP domains removed) *Lactobacillus crispatus* fructosidase of SEQ ID NO:3, comprising the mutation T674V.

SEQ ID NO: 8 LF3GS, amino acid sequence of a truncated (SLAP, LamG, and Big2 domains removed) *Lactobacillus crispatus* fructosidase containing the amino acids 1-859 and comprising the mutation T674A.

SEQ ID NO: 9 LFM24, amino acid sequence of a full-length wild type *Lactobacillus amylovorus* fructosidase without signal peptide, containing the amino acids 1-1259.

SEQ ID NO: 10 LFM24S, amino acid sequence of a truncated (SLAP domain removed) *Lactobacillus amylovorus* fructosidase containing the amino acids 1-1145 of the LFM24.

SEQ ID NO: 11 LFM24GS, amino acid sequence of a truncated (SLAP domain removed) *Lactobacillus amylovorus* fructosidase, containing the amino acids 1-1145 of the LFM24 and a mutation T655A.

SEQ ID NO: 12 LFM33, amino acid sequence of a full-length wild type *Lactobacillus crispatus* fructosidase without signal peptide, containing the amino acids 1-1279.

SEQ ID NO: 13 LFM33GS, amino acid sequence of a truncated (SLAP domain removed) *Lactobacillus crispatus* fructosidase containing the amino acids 1-1165 of the LFM33 and a mutation T674A.

**DETAILED DESCRIPTION**

In the following description, like reference signs denote like elements or steps.

As used herein, the term "fructanase" or "fructosidase" or "exo-beta-D-fructosidase" refers to fructan beta-fructosidase, which is an enzyme with systematic name beta-D-fructan fructohydrolase (EC 3.2.1.80). Fructanase catalyzes the hydrolysis of terminal, non-reducing (2->1)- and (2->6)-linked beta-D-fructofuranose residues in fructans. Substrates of fructanase include inulin, levan and sucrose. Fructanase degrades levans and inulins to fructose and also cleaves sucrose into glucose and fructose and can therefore function as an extracellular invertase.

As used herein, the term "variant polypeptide" refers to a variant which is a polypeptide. The term "variant polypeptide" used herein may refer to the variant polypeptide of the first aspect, or to another variant polypeptide. As used herein, the term "variant" means a sequence or a fragment of a sequence (nucleotide or amino acid) inserted, substituted, or deleted by one or more nucleotides/amino acids, or which is chemically modified. The term variant may in some embodiments also include the variant polypeptide (of fructanase), and/or the fusion protein including the variant polypeptide (of fructanase).

As used herein, the term "fructanase variant" and "variant fructanase" and "variant of fructanase" refers to a fructanase molecule obtained by site-directed or random mutagenesis, insertion, substitution, deletion, recombination and/or any other suitable protein engineering method, and which leads into a genetically modified fructanase variant that differs in its amino acid sequence from the parent molecule/ fructanase such as a wild type fructanase. The terms "wild type fructanase", "wild type enzyme", "wild type", or "wt" in accordance with the disclosure, describe a fructanase enzyme with an amino acid sequence found in nature or a fragment thereof. The variant encoding gene can be synthesized, or the parent gene be modified using genetic methods, e.g., by site directed mutagenesis, a technique in which one or more than one mutation are introduced at one or more defined sites in a polynucleotide encoding the parent polypeptide.

The term "parent fructanase polypeptide" or "wild type parent fructanase" or "wild type parent polypeptide" means a fructanase polypeptide, from which a specific variant fructanase polypeptide is derived from.

The term "variant polypeptide" of fructanase according to the first aspect, may also be referred to by using the name given to variant, e.g., LF2-LF5.

As used herein, the term "GH32" or the "glycosyl hydrolase 32" refers to a family of enzymes that hydrolyze fructose-containing polysaccharides, comprising at least inulinases, exoinulinases, levanases, 2,6-β-fructan 6-levanbiohydrolases, fructan β-(2,1)-fructosidases and fructan β-(2,6)-fructosidases.

The term "GH32 domain" refers to the catalytic domain of the glycosyl hydrolase 32 enzymes. For estimating the amino acid region comprising the GH32 domain for each fructanase polypeptide, the ColabFold web tool can be used. For example, the ColabFold web tool was used to predict the structure of *Lactobacillus crispatus* fructosidase SEQ ID NO: 1. and based on the obtained results the catalytic domain GH32 of the SEQ ID NO: 1 comprises the amino acids 383 - 858.

ColabFold is a web-based implementation of the AlphaFold2 protein folding algorithm, capable of predicting the 3D structure of a protein from its amino acid sequence (Mirdita et al., 2022). ColabFold runs on Google Colab, a cloud-based platform for running Jupyter notebooks. Instructions provided in the notebook are used to upload protein sequence in FASTA format. The ColabFold tool performs the following steps: 1) Pre-processing: The input sequence is checked for errors and inconsistencies. 2) Fold recognition: ColabFold first searches a database of known protein structures to identify proteins with similar sequences to input sequence. This information found in the search is used to guide the subsequent structure prediction process. 3) Structure prediction: Using the AlphaFold2 algorithm, ColabFold predicts the 3D structure of protein. The predicted structure is output in PDB format. ColabFold website: (https://colab.research.google.com/github/sokrypton/ColabFold/blob/main/AlphaFold2.ipyn b).

As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues.

As used herein, the term "mature polypeptide" means any polypeptide wherein at least one signal sequence or signal peptide or signal peptide and a putative pro-peptide, or a carrier peptide or a fusion partner is cleaved off. For example, the "mature polypeptide" of variant LF2 comprises the amino acids 1-1165 of SEQ ID NO:1.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native, or it can be obtained from another source. Depending on the host cell, the larger polypeptide may be cleaved from the secretory peptide during transit through the secretory pathway, thereby forming a mature polypeptide lacking the secretory peptide.

As used herein, a "protein" may refer to a peptide or a polypeptide of any size. A protein may be an enzyme, a protein, an antibody, a membrane protein, a peptide hormone, regulator, or any other protein.

As used herein, "substitution" or "amino acid substitution" means an amino acid residue replacement with an amino acid residue that is different than the original amino acid in that specific replacement position. The term "amino acid substitution" can refer to both, conservative amino acid substitutions and non-conservative amino acid substitutions, which means the amino acid residue is replaced with an amino acid residue having a similar side chain (conservative), or a different side chain (non-conservative), as the original amino acid residue in that place. Substitutions are described using the following nomenclature: amino acid residue in the protein scaffold; position; substituted amino acid residue(s). According to this nomenclature the substitution of, for instance, a single residue of Threonine to Alanine residue at position 674 is indicated as Thr674Ala or T674A.

By "N-glycosylation" is meant attachment of an oligosaccharide to a nitrogen atom, usually to the nitrogen atom of asparagine residue that is present as a part of Asn-Xaa-Ser/Thr (N-Xaa-S/T) consensus sequence, where Xaa is any amino acid except proline (Pro) present in the polypeptide in question. As used herein, the term "N-glycosylation site" refers to a site in an amino acid chain, wherein the amino acid at that site can be N-glycosylated.

As used herein, "host cell" means any cell type that is susceptible to transformation, transfection, transduction, mating, crossing, CRISPR-Cas or the like with a nucleic acid construct or expression vector comprising a polynucleotide. The term "host cell" encompasses any progeny that is not identical due to mutations that occur during replication. Non-limiting examples of a host cell are fungal cells, preferably a filamentous fungal cell (e.g. *Trichoderma* or *Trichoderma reesei, Aspergillus* or *Aspergillus oryzae* or A. *niger, Thermothelomyces* or *Thermothelomyces heterothallica, Myceliophthora* or *Myceliophthora thermophila, Humicola* or *Humicola insolens, Fusarium* or *Fusarium venenatum*), and bacterial cells, such as gram-positive Bacilli (e.g., *Bacillus subtilis, Bacillus pumilus, Alkalihalobacillus alcalophilus, Bacillus amyloliquefaciens, Bacillus velezensis, Brevibacillus brevis, Niallia circulans, Shouchella clausii, Weizmannia coagulans, Halalkalibacterium halodurans, Paenibacillus lautus, Lederbergia lenta, Bacillus licheniformis, Bacillus paralicheniformis Priestia megaterium, Geobacillus stearothermophilus, or Bacillus thuringiensis*), and gram-negative bacteria (e.g., *Escherichia coli*), actinomycetales (e.g., *Streptomyces* sp., *Nonomuraea flexuosa*) and yeasts (e.g., *Saccharomyces cerevisiae, Schizosaccharomyces pombe, Pichia pastoris, Yarrowia lipolytica*).

As used herein, "sequence identity" means the percentage of exact matches of nucleotides or amino acid residues between two optimally aligned sequences over the number of positions where there are nucleotides/residues present in both sequences. When one sequence has a residue with no corresponding residue in the other sequence, the alignment program allows a gap in the alignment, and that position is not counted in the denominator of the identity calculation. For purposes of the present invention, the sequence identity between two amino acid sequences can be determined using the Needleman-Wunsch algorithm (Needleman et al. 1970) as implemented in the Needle program of the EMBOSS package (Rice et al., 2000), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment}).$$

As used herein, "sequence alignment" of the amino acid sequences means, aligning the sequences using Clustal Omega (1.2.4) multiple sequence alignment program (https://www.ebi.ac.uk/Tools/msa/clustalo/) as described by Sievers et al. 2011, and using the default settings.

As used herein, the term "corresponding positions" or "corresponding amino acid position" or "corresponding amino acids" means aligning at least two amino acid sequences according to identified regions of similarity or identity as pairwise alignment or as multiple sequence alignment, thereby pairing up the corresponding amino acids. In the present disclosure "a corresponding position" typically refers to a position corresponding to a position in SEQ ID NO: 1. The corresponding amino acids between two amino acid sequences need not, but can be, the same amino acids.

Unless otherwise specified, all references to a certain amino acid position refer to an amino acid of the SEQ ID NO: 1 in said position, or to an amino acid present or missing in the corresponding position of an amino acid sequence aligned with SEQ ID NO: 1.

As used herein, the term "fructanase productivity" refers to a level of fructanase production by means of transcription, translation, post-translational modifications and ultimately intracellular or extracellular targeting and/or secretion of the fructanase. Fructanase productivity can thereby refer to the intracellular level of translated fructanase or indicate the level of extracellularly secreted fructanase. Methods for protein production by recombinant technology in different host systems are known in the art. Preferably, the fructanase variants are produced as extracellular proteins that are secreted into the culture medium wherein the host cell is cultured, and from which they can be easily recovered and isolated.

As used herein, with "fructanase activity" or "fructan degrading performance" is meant the ability of fructanase to catalyze the hydrolysis of terminal, non-reducing (2->1)- and (2->6)-linked beta-D-fructofuranose residues into fructans. The fructanase activity can be indicated as relative fructanase activity, wherein fructanase activity of a sample is indicated as a fold change or as a percentage in relation to the control sample. Example 3 provides an example of a method for determining fructanase activity. The fructanase activity can also be indicated in enzymatic units/ml (U/ml), wherein one enzymatic unit is defined as the amount of the enzyme which produces 1 $\mu$mol of reducing sugars per ml.

As used herein, the term S-layer associated protein" or "SLAP" domain refers to amino acid sequence homologous to short domain which is found in a variety of bacterial cell surface proteins. As used herein, the term "SLAP domain" can in specific embodiments refer to a SLAP domain belonging to a Pfam family PF03217, i.e., a SLAP domain according to EMBL-EBI PFAM 35.0 database and hidden Markov model profiles (HMM profiles).

As used herein, the "PFAM database", such as PFAM 35.0 database, refers to a database of protein families that includes their annotations and multiple sequence alignments generated using hidden Markov models. In this context is meant the version PFAM 35.0 from November 2021 of the PFAM database, available for all computer platforms from e.g., https://pfam.xfam.org/.

As used herein, the term "HMM profile" refers to hidden Markov model profiles, which are computational algorithms/models used for predicting protein structure and function, which identify significant protein sequence similarities allowing the detection of homologs. HMM profiles also allow encapsulating the evolutionary changes that have occurred in a set of related sequences (i.e., a multiple sequence alignment). HMM profiles capture position-specific information about how conserved each amino acid is in each column of the alignment. HMM profiles may be generated online, and the HMM profiles may be obtained from the PFAM database. HMM profiles may be generated also locally on a computer using the HMMER software package (current version HMMER 3.3.2 from December 2021). HMMER database is available for all computer platforms from http://hmmer.org/.https://pfam.xfam.org/.

As used herein, the term "C-terminal" or "carboxy-terminal" refers to the end of an amino acid chain (protein, polypeptide, peptide), terminated by a free carboxyl group -COOH. With the term "C-terminal amino acid sequence" is meant an amino acid sequence from which the majority of is located at the C-terminal half of the entire amino acid sequence in question.

As used herein, the term "N-terminal" or "amino-terminal" refers to the end of an amino acid chain (protein, polypeptide or peptide), starting with a free amine group ($-NH_2$). With the term "N-terminal amino acid sequence" is meant an amino acid sequence from which the majority of is located at the N-terminal half of the entire amino acid sequence in question.

As used herein, the term "plant-based material" refers to materials and products that are partially or wholly derived from plants.

With the term "enzyme composition" is meant an enzymatic fermentation product, usually cell-free and possibly further isolated and purified, typically produced by a pure culture of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques. The enzyme composition may contain, for example, stabilators and preservatives which prevent microbial growth and improve storage stability. In an embodiment the term "enzyme composition" means a composition obtained by steps of culturing a host cell and either recovering at least one enzyme, which may be a polypeptide, from the cells or separating the cells from the spent culture medium and obtaining the supernatant comprising the at least one enzyme. The spent culture medium of the production host can be used as such, or the host cells may be removed, and/or it may be concentrated, filtrated or fractionated. It may also be dried. The obtained enzyme composition may be in the form of liquid, flour, powder, granulate or tablets. The enzyme may be in immobilized form in the composition. The enzyme composition may be a monocomponent, or have other enzymes produced by the production host, or a mixture of enzymes from different sources.

The term "carrier" or "carrier polypeptide" refers to a polypeptide into which the protein of interest (fructanase) is translationally fused to improve the yield. The carrier can be either homologous or heterologous to the production host in its origin and can be a full-length protein or a fragment of a protein (e.g., a core, a CBM or a CBM and linker region). It is preferably encoded by a gene or a nucleotide sequence with good expression level.

As used herein, the term "expression" includes any step or all steps involved in the production of a polypeptide in a host cell including, but not limited to, transcription, translation, post-translational modification, and secretion. Expression may be followed by harvesting, i.e., recovering, the host cells or the expressed product.

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | lie | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| X | Xaa | Any amino acid |

As used herein, the term "comprising" includes the broader meanings of "including", "containing", and "comprehending", as well as the narrower expressions "consisting of" and "consisting only of".

In an embodiment, a variant polypeptide has: fructanase activity; an amino acid sequence having at least 80 % amino acid sequence identity with the amino acids 383 - 858 of SEQ ID NO: 1; and at least one substitution, insertion or deletion of an amino acid, the at least one substitution, insertion or deletion preventing N-glycosylation of an amino acid at the amino acid position 672 of the variant polypeptide when expressed in a host cell capable of N-glycosylation, wherein the amino acid numbering of the amino acid having N-glycosylation prevented corresponds to the amino acid numbering of the SEQ ID NO: 1. In an embodiment, the amino acid numbering of the at least one substitution, insertion or deletion of an amino acid of the variant polypeptide corresponds to the amino acid numbering of the SEQ ID NO: 1. In an embodiment, the amino acid numbering of the variant polypeptide corresponds to the amino acid numbering of the SEQ ID NO: 1.

In an embodiment, the amino acid of the variant polypeptide having N-glycosylation prevented by the at least one substitution, insertion or deletion of an amino acid, and corresponding to the amino acid position 672 of SEQ ID NO:1, is asparagine (N).

In an embodiment, the at least one substitution, insertion or deletion of an amino acid prevents N-glycosylation of asparagine (N) present in the wild type parent polypeptide of the variant polypeptide, but which is not necessarily present in the variant polypeptide.

In an embodiment, the amino acid of the variant polypeptide having N-glycosylation prevented by the at least one substitution, insertion or deletion of an amino acid, and corresponding to the amino acid position 672 of SEQ ID NO:1, is another amino acid than asparagine (N). In an embodiment, the variant polypeptide has at least one substitution of an amino acid at the amino acid position corresponding to the amino acid position 672 of SEQ ID NO:1, wherein the substituent amino acid is not asparagine (N). In an embodiment, the variant polypeptide has at least one deletion of an amino acid at the amino acid position corresponding to the amino acid position 672 of SEQ ID NO:1, wherein the deleted amino acid is asparagine (N).

In an embodiment, the variant polypeptide has fructanase activity; an amino acid sequence having at least 80 % amino acid sequence identity with the amino acids 383 - 858 of SEQ ID NO: 1; and at least one substitution, insertion or deletion of an amino acid, the at least one substitution, insertion or deletion preventing N-glycosylation of asparagine (N) at an amino acid position corresponding to the amino acid position 672 of SEQ ID NO:1, when expressed in a host cell capable of N-glycosylation.

SEQ ID NO:1 is LFRU, indicating a full-length *Lactobacillus crispatus* fructosidase amino acid sequence without a signal peptide, comprising 1279 amino acids. In an embodiment, the variant polypeptide is an extracellular fructosidase and a variant of glycosyl hydrolase family 32 (GH32) family member, wherein the variant polypeptide has fructanase activity. The catalytic domain GH32 of the SEQ ID NO: 1 comprises the amino acids 383 - 858. In an embodiment, the variant polypeptide has an amino acid sequence having a high, such as at least 80 %, sequence identity with the amino acids comprised by the GH32 domain of the SEQ ID NO: 1. In an embodiment, the variant polypeptide is a GH32 family fructanase which may have endo and/or exo activity.

In an embodiment, the amino acid sequence of the variant polypeptide has at least 80 %, but less than 100 % amino acid sequence identity with amino acids 383 - 858 of SEQ ID NO: 1; and at least one substitution, insertion or deletion of an amino acid, the at least one substitution, insertion or deletion preventing N-glycosylation of an amino acid at the amino acid position corresponding to the amino acid position 672 of the of the SEQ ID NO: 1. In an embodiment, the variant polypeptide comprises at least one further substitution, insertion or deletion of an amino acid preventing N-glycosylation at a different amino acid position of the variant polypeptide than at the position corresponding to the amino acid position 672 of the of the SEQ ID NO: 1, when expressed in a host cell capable of N-glycosylation.

In an embodiment, the amino acid sequence of the variant polypeptide has at least one substitution of an amino acid preventing N-glycosylation of an amino acid at the amino acid position corresponding to the amino acid position 672 of the of the SEQ ID NO: 1. In an embodiment, the amino acid sequence of the variant polypeptide has at least one insertion of an amino acid preventing N-glycosylation of an amino acid at the amino acid position corresponding to the amino acid position 672 of the of the SEQ ID NO: 1. In an embodiment, the amino acid sequence of the variant polypeptide has at least one deletion of an amino acid preventing N-glycosylation of an amino acid at the amino acid position corresponding to the amino acid position 672 of the of the SEQ ID NO: 1.

In an embodiment, the at least one substitution, insertion or deletion of an amino acid prevents the N-glycosylation of an amino acid at the amino acid position of the variant polypeptide corresponding to the amino acid position 672 of the SEQ ID NO: 1 only when expressed in a host cell capable of N-glycosylation. Cells capable of N-glycosylation generally comprise eukaryotes and archaea, whereas bacterial cells are rarely capable of N-glycosylation, although some bacterial cells can link glycans to amide nitrogens of asparagines.

In an embodiment, the at least one amino acid substitution, insertion or deletion comprised by the amino acid sequence of the variant polypeptide destroys a consensus sequence N-X-S/T for N-glycosylation, wherein X is any amino acid except proline (Pro).

In an embodiment, the variant polypeptide comprises the at least one substitution, insertion or deletion within the consensus sequence N-X-S/T for N-glycosylation, the at least one substitution, insertion or deletion preventing N-glycosylation of an amino acid at the amino acid position corresponding to the amino acid position 672 of the of the SEQ ID NO: 1.

In an embodiment, the amino acid sequence of the variant polypeptide does not comprise the consensus sequence for N-glycosylation N-X-S/T at the amino acid positions corresponding to the amino acid positions 672- 674 of the of the SEQ ID NO: 1.

In an embodiment, the variant polypeptide has at least one substitution, insertion and deletion of an amino acid.

In an embodiment, the at least one amino acid substitution or deletion of the variant polypeptide is at amino acid position 672, 673, 674, or any combination thereof. In such an embodiment, the amino acid numbering of the at least one amino acid substitution or deletion corresponds to the amino acid numbering of the SEQ ID NO: 1.

In an embodiment, the at least one amino acid substitution or deletion of the variant polypeptide is at amino acid position corresponding to amino acid position 672, 673, or 674 of the SEQ ID NO: 1, or any combination thereof.

In an embodiment, the variant polypeptide comprises at least one amino acid substitution which is at amino acid position corresponding to the amino acid positions 672, 673, or 674 of the SEQ ID NO: 1, or any combination thereof. In an embodiment, the variant polypeptide comprises at least one amino acid deletion which is at amino acid position corresponding to the amino acid position 672, 673, or 674 of the SEQ ID NO: 1, or any combination thereof. In an embodiment, the variant polypeptide comprises at least one amino acid substitution and at least one deletion at amino acid positions selected from the positions corresponding to the amino acid positions 672, 673, and 674 of the SEQ ID NO: 1, and any combination thereof.

In an embodiment, the variant polypeptide comprises the amino acid substitutions at the amino acid positions corresponding to the amino acid positions 672 and 673 and 674 of the SEQ ID NO: 1. In an embodiment, the variant polypeptide comprises the amino acid substitutions at the amino acid positions corresponding to the amino acid positions 672 and 673, or positions 673 and 674, or positions 672 and 674 of the SEQ ID NO: 1. Preferably the variant polypeptide comprises the amino acid substitution at least at the amino acid position corresponding to the amino acid position 674 or 672 of the SEQ ID NO: 1, preferably corresponding to the amino acid position 674 of the SEQ ID NO: 1.

In an embodiment, the variant polypeptide comprises the amino acid deletion at amino acid positions corresponding to the amino acid positions 672 and 673 and 674 of the SEQ ID NO: 1. In an embodiment, the variant polypeptide comprises the amino acid deletions at amino acid positions corresponding to the amino acid positions 672 and 673, or positions 673 and 674, or positions 672 and 674, of the SEQ ID NO: 1. Preferably the variant polypeptide comprises the amino acid deletion at least at the amino acid position corresponding to the amino acid position 674 or 672 of the SEQ ID NO: 1, preferably at the amino acid position corresponding to the amino acid position 674 of the SEQ ID NO: 1.

In an embodiment, the at least one insertion of an amino acid of the variant polypeptide is between the amino acids 672 and 673, or between the amino acids 673 and 674. In such an embodiment, the numbering of the amino acid positions between which the at least one amino acid insertion is located, corresponds to the amino acid numbering of the SEQ ID NO: 1.

In an embodiment, the at least one insertion of an amino acid of the variant polypeptide is between the amino acids corresponding to the amino acids 672 and 673 of the SEQ ID NO: 1, or between the amino acids corresponding to the amino acids 673 and 674 of the SEQ ID NO: 1.

In a preferable embodiment, the at least one insertion of an amino acid between the amino acids corresponding to the amino acids 672 and 673 of the SEQ ID NO: 1, or between the amino acids corresponding to the amino acids 673 and 674 of the SEQ ID NO: 1, is an insertion of one amino acid. In some other embodiments, the at least one insertion of an amino acid between the amino acids corresponding to the amino acids 672 and 673 of the SEQ ID NO: 1, or between the amino acids corresponding to the amino acids 673 and 674 of the SEQ ID NO: 1, is an insertion of more than one amino acid, such as two or three amino acids.

In an embodiment, the variant polypeptide comprises an amino acid insertion between the amino acids corresponding to the amino acids 672 and 673 of the SEQ ID NO: 1, and also between the amino acids corresponding to the amino acids 673 and 674 of the SEQ ID NO: 1. In an embodiment, the variant polypeptide comprises an amino acid insertion between the amino acids 672 and 673, and/or between the amino acids 673 and 674, wherein the amino acid numbering of the amino acid positions corresponds to the amino acid numbering of the SEQ ID NO: 1.

In an embodiment, the at least one amino acid substitution or deletion of the variant polypeptide is at amino acid position N672, G673, T674, or any combination thereof; and/or the at least one amino acid insertion of the variant polypeptide is between amino acid positions N672 and G673, or between amino acid positions G673 and T674; wherein the amino acid numbering of the amino acid positions corresponds to the amino acid numbering of SEQ ID NO:1.

In an embodiment, the at least one amino acid substitution or deletion of the variant polypeptide is at amino acid position corresponding to the amino acid position N672, G673, or T674 of the SEQ ID NO: 1, or any combination thereof; and/or the at least one amino acid insertion of the variant polypeptide is between amino acid positions corresponding to the amino acid positions N672 and G673 of the SEQ ID NO: 1, or between amino acid positions corresponding to the amino acid position G673 and T674 of the SEQ ID NO: 1.

It is to be understood, that in the following embodiments, wherein the number of the mutated amino acid and the specific amino acid at that site (to be mutated) is specified, the amino acid numbering of the mutated amino acids of the variant polypeptide corresponds to the amino acid numbering of the SEQ ID NO: 1. In an embodiment, the variant polypeptide comprises at least one amino acid substitution at amino acid position N672, G673, T674, or any combination thereof. In an embodiment, the variant polypeptide comprises amino acid substitutions at amino acid positions N672 and G673. In an embodiment, the variant polypeptide comprises amino acid substitutions at amino acid positions N672 and T674. In an embodiment, the variant polypeptide comprises amino acid substitutions at amino acid positions G673 and T674. In an embodiment, the variant polypeptide comprises amino acid substitutions at amino acid positions N672 and G673 and T674.

In an embodiment, the variant polypeptide comprises at least one amino acid deletion at amino acid position N672, G673, T674, or any combination thereof. In an embodiment, the variant polypeptide comprises amino acid deletions at amino acid positions N672 and G673. In an embodiment, the variant polypeptide comprises amino acid deletions at amino acid positions N672 and T674. In an embodiment, the variant polypeptide comprises amino acid deletions at amino acid positions G673 and T674. In an embodiment, the variant polypeptide comprises amino acid deletions at amino acid positions N672 and G673 and T674.

In an embodiment, the variant polypeptide comprises at least one amino acid substitution and at least one amino acid deletion at amino acid positions selected from the positions N672, G673, T674. In an embodiment, the variant polypeptide comprises an amino acid substitution and/or an amino acid deletion at each of the amino acid positions N672, G673, T674.

In an embodiment, the variant polypeptide comprises at least one insertion of an amino acid between the amino acids N672 and G673, or between the amino acids G673 and T674. In an embodiment, the variant polypeptide comprises at least one insertion of an amino acid between the amino acids N672 and G673. In an embodiment, the variant polypeptide comprises at least one insertion of an amino acid between the amino acids G673 and T674. In an embodiment, the variant polypeptide comprises an insertion of at least one amino acid between the amino acids N672 and G673, and between the amino acids G673 and T674.

In an embodiment, the variant polypeptide comprises at least one amino acid substitution at amino acid position N672, G673, T674, or any combination thereof, and at least one insertion of an amino acid between the amino acids N672 and G673, or between the amino acids G673 and T674. In an embodiment, the variant polypeptide comprises at least one amino acid deletion at amino acid position N672, G673, T674, or any combination thereof, and at least one insertion of an amino acid between the amino acids N672 and G673, or between the amino acids G673 and T674.

In an embodiment, the variant polypeptide comprises an amino acid substitution at the amino acid position N672, wherein the N672 is substituted with any other amino acid than Asparagine (N). In an embodiment, the variant polypeptide comprises an amino acid substitution at the amino acid position G673, wherein the G673 is substituted with Proline (P). In an embodiment, the variant polypeptide comprises an amino acid substitution at the amino acid position T674, wherein the T674 is substituted with any other amino acid than Threonine (T) or Serine (S).

In an embodiment, the at least one amino acid substitution of the variant polypeptide is selected from N672Q, G673P, T674A, T674G, and T674V, or combinations thereof, wherein the amino acid numbering of the amino acid substitutions corresponds to the amino acid numbering of SEQ ID NO: 1.

It is to be understood, that in the following embodiments, wherein the number of the substituted amino acid(s), and the specific substituted amino acid(s) and substituent amino acid(s) at that site are specified, the amino acid numbering of the substituted amino acid of the variant polypeptide corresponds to the amino acid numbering of the SEQ ID NO: 1.

In an embodiment, the variant polypeptide comprises the amino acid substitution N672Q. In an embodiment, the variant polypeptide comprises the amino acid substitution G673P. In an embodiment, the variant polypeptide comprises the amino acid substitution T674A. In an embodiment, the variant polypeptide comprises the amino acid substitution T674G. In an embodiment, the variant polypeptide comprises the amino acid substitution T674V.

In an embodiment, the variant polypeptide comprises the amino acid substitutions N672Q and G673P. In an embodiment, the variant polypeptide comprises the amino acid substitutions N672Q and T674A. In an embodiment, the variant polypeptide comprises the amino acid substitutions G673P and T674A.

In an embodiment, the variant polypeptide comprises the amino acid substitutions N672Q and T674G. In an embodiment, the variant polypeptide comprises the amino acid substitutions G673P and T674G. In an embodiment, the variant polypeptide comprises the amino acid substitutions N672Q and T674V. In an embodiment, the variant polypeptide comprises the amino acid substitutions G673P and T674V.

In an embodiment, the variant polypeptide comprises the amino acid substitutions N672Q, G673P and T674A. In an embodiment, the variant polypeptide comprises the amino acid substitutions N672Q, G673P and T674G. In an embodiment, the variant polypeptide comprises the amino acid substitutions N672Q, G673P and T674V.

In an embodiment, the variant polypeptide comprises an insertion of at least one amino acid between the amino acids N672 and G673, wherein the inserted at least one amino acid can be any amino acid. In an embodiment, the variant polypeptide comprises an insertion of at least one amino acid between the amino acids G673 and T674, wherein the inserted at least one amino acid can be any other amino acid than Threonine (T) or Serine (S).

In an embodiment, the variant polypeptide may comprise any insertion of at least one amino acid between the amino acids N672 and G673, and/or between the amino acids G673 and T674, as long as the resulting variant polypeptide has fructanase activity and the insertion of at least one amino acid destroys a consensus sequence N-X-S/T for N-glycosylation.

In an embodiment, the variant polypeptide having the at least one substitution, insertion or deletion of an amino acid, the at least one substitution, insertion or deletion preventing N-glycosylation of an amino acid at the amino acid position corresponding to the amino acid position 672 of the SEQ ID NO: 1, when expressed in a host cell capable of N-glycosylation, is beneficial as it has an increased fructanase productivity and/or improved fructan degrading performance, when compared to a polypeptide having the sequence SEQ ID NO: 1, preferably, the variant polypeptide has at least an improved fructan degrading performance.

In an embodiment, the size of the variant polypeptide is at least 476 amino acids.

In an embodiment, the size of the variant polypeptide is 476 - 1279 amino acids, 476 - 1223 amino acids, 476 - 1165 amino acids, 476 - 964 amino acids, 476 - 867 amino acids, 476 - 858 amino acids, 476 - 590 amino acids, or 476 - 482 amino acids, preferably the variant polypeptide is 476 - 1165 amino acids in size. In such an embodiment, the size of the variant polypeptide is given in respect of a variant polypeptide having the at least amino acid substitution preventing N-glycosylation of an amino acid at the amino acid position corresponding to the amino acid position 672 of the SEQ ID NO: 1, when expressed in a host cell capable of N-glycosylation. It is however, understood that if the variant polypeptide comprises the at least one amino acid deletion and/or the at least amino acid insertion, the size of the variant changes accordingly. In an example embodiment, the amino acid sequence of the variant polypeptide comprises the at least one amino acid deletion, and the size of the variant polypeptide is at least 475 amino acids. In another example embodiment, wherein the amino acid sequence of the variant polypeptide comprises more than one amino acid deletions, such as two or three deletions, the size of the variant polypeptide can be at least 474 or at least 473 amino acids, respectively. In a further example embodiment, the amino acid sequence of the variant polypeptide comprises the GH32 domain and at least one amino acid insertion, and the size of the variant polypeptide is at least 477 amino acids. In some embodiments, wherein the amino acid sequence of the variant polypeptide comprises more than one amino acid insertions, such as two insertions, the size of the variant polypeptide can be at least 478 amino acids, respectively. In an embodiment, the size of the variant polypeptide is 478 - 1281 amino acids.

In an embodiment, the variant polypeptide comprises at least a GH32 domain, and the size of the variant polypeptide is at least 476 amino acids. In an embodiment, the amino acid sequence of the variant polypeptide comprises the at least one amino acid substitution, and the size of the variant polypeptide is at least 476 amino acids. In an embodiment, the size of the variant polypeptide is 476 - 1279 amino acids.

In an embodiment, the amino acid sequence of the variant polypeptide consists only of a GH32 domain. In an embodiment, the amino acid sequence of the variant polypeptide comprises a GH32 domain, and at least one domain selected from: one or two LamG domains, a Cadherine-like domain, Big-2 domain, one or two SLAP domains, or any combination thereof (according to Fig. 1).

In an embodiment, the size of the variant polypeptide is 1223 amino acids or less, 1220 amino acids or less, or 1225 amino acids or less, and it only comprises one SLAP domain.

In an embodiment, the size of the variant polypeptide is 1165 amino acids or less, 1162 amino acids or less, or 1167 amino acids or less, and it is obtained via a C-terminal deletion of SLAP domain amino acids 1166-1276, the amino acid numbering of the deleted amino acids corresponding to the amino acid numbering of the SEQ ID NO: 1.

In an embodiment, the size of the variant polypeptide is 964 amino acids or less, 961 amino acids or less, or 966 amino acids or less, and it is obtained via a C-terminal deletion of LamG and SLAP domain amino acids 965 - 1276, the amino acid numbering of the deleted amino acids corresponding to the amino acid numbering of the SEQ ID NO: 1.

In an embodiment, the size of the variant polypeptide is 867 amino acids or less, 864 amino acids or less, or 869 amino acids or less, and it is obtained via a C-terminal deletion of Big-2, LamG and SLAP domain amino acids 868 - 1276, the amino acid numbering of the deleted amino acids corresponding to the amino acid numbering of the SEQ ID NO: 1.

In an embodiment, the size of the variant polypeptide is 858 amino acids or less, 855 amino acids or less, or 860 amino acids or less, and it is obtained via a C-terminal deletion of Big-2, LamG and SLAP domain amino acids 859 - 1276, the amino acid numbering of the deleted amino acids corresponding to the amino acid numbering of the SEQ ID NO: 1.

In an embodiment, the size of the variant polypeptide is 599 amino acids or less, 596 amino acids or less, or 601 amino acids or less, and it is obtained via a C-terminal deletion of Big-2, LamG and SLAP domain amino acids 868 - 1276 and an N-terminal deletion of LamG domain amino acids 1-268, the amino acid numbering of the deleted amino acids corresponding to the amino acid numbering of the SEQ ID NO: 1.

In an embodiment, the size of the variant polypeptide is 491 amino acids or less, 488 amino acids or less, or 493 amino acids or less, and it is obtained via a C-terminal deletion of Big-2, LamG and SLAP domain amino acids 868 - 1276 and an N-terminal deletion of LamG and Cadherine-like domain amino acids 1-376, the amino acid numbering of the deleted amino acids corresponding to the amino acid numbering of the SEQ ID NO: 1.

In an embodiment, the variant polypeptide having the size of at least 476 amino acids, but less than 1279 amino acids is beneficial as it has an increased fructanase productivity, when compared to a polypeptide having the sequence SEQ ID NO: 1, preferably the size of the variant polypeptide is 476 - 1165 amino acids.

In an embodiment, the variant polypeptide has amino acids corresponding to amino acids 1 - 1279 of the SEQ ID NO: 1 or less, amino acids 1 - 1223 or less, amino acids 1 - 1165 or less, amino acids 1 - 964 or less, amino acids 1 - 867 or less, amino acids 1 - 858 or less, amino acids 269 - 858 or less, or amino acids 377 - 858 or less.

In an embodiment, the variant polypeptide has amino acids corresponding to amino acids from 1 to 1279 of the SEQ ID NO: 1 or less. In an embodiment, the variant polypeptide has amino acids corresponding to amino acids from 1 to 1223 of the SEQ ID NO: 1 or less. In an embodiment, the variant polypeptide has amino acids corresponding to amino acids from 1 to 1165 of the SEQ ID NO: 1 or less. In an embodiment, the variant polypeptide has amino acids corresponding to amino acids from 1 to 964 of the SEQ ID NO: 1 or less. In an embodiment, the variant polypeptide has amino acids corresponding to amino acids from 1 to 867 of the SEQ ID NO: 1 or less. In an embodiment, the variant polypeptide has amino acids corresponding to amino acids from 1 to 858 of the SEQ ID NO: 1 or less. In an embodiment, the variant polypeptide has amino acids corresponding to amino acids from 269 to 858 of the SEQ ID NO: 1 or less. In an embodiment, the variant polypeptide has amino acids corresponding to amino acids from 377 to 858 of the SEQ ID NO: 1 or less.

In an embodiment, the variant polypeptide has amino acids corresponding to amino acids 1 - 1279 of the SEQ ID NO: 1, or to amino acids 1 - 1223 of the SEQ ID NO: 1, or to amino acids 1 - 1165 of the SEQ ID NO: 1, or to amino acids 1 - 964 of the SEQ ID NO: 1, or to amino acids 1 - 867 of the SEQ ID NO: 1, or to amino acids 1 - 858 of the SEQ ID NO: 1, or to amino acids 269 - 858 of the SEQ ID NO: 1, or to amino acids 377 - 858 of the SEQ ID NO: 1.

In a preferred embodiment, the variant polypeptide has at least amino acids corresponding to the amino acids from 383 to 858 of the SEQ ID NO: 1. In a further preferred embodiment, the variant polypeptide has at least amino acids corresponding to the amino acids from 383 to 858 of the SEQ ID NO: 1, and it is obtained via a C-terminal deletion of SLAP domain amino acids corresponding to the amino acids 1166-1276 of the SEQ ID NO: 1. In a further preferred embodiment, the variant polypeptide is without SLAP domain amino acids corresponding to the amino acids 1166-1276 of the SEQ ID NO: 1. In a further preferred embodiment, the variant polypeptide has at least the amino acids corresponding to the amino acids from 1 to 858 of the SEQ ID NO: 1 and the variant polypeptide is without SLAP domain amino acids corresponding to the amino acids 1166-1276 of the SEQ ID NO: 1.

In an embodiment, the variant polypeptide has at least 85 %, preferably at least 90 %, more preferably at least 95 %, even more preferably at least 98 % amino acid sequence identity with the amino acids 383 - 858 of SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 278 - 858 of the SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 96 - 858 of the SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 1 - 858 of the SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 1 - 953 of the SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 1 - 1118 of the SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 1 - 1216 of the SEQ ID NO: 1.

In an embodiment, the variant polypeptide has an amino acid sequence having at least 85 %, preferably at least 90 % more preferably at least 95 % even more preferably at least 98 % amino acid sequence identity with the amino acids 1 - 1279 of the SEQ ID NO: 1.

In an embodiment, the variant polypeptide has fructanase activity; an amino acid sequence having at least 80 % amino acid sequence identity with the amino acids 383 - 858, or with the amino acids 1- 858, or with the amino acids 1 - 953, or with the amino acids 1 - 1118, or with the amino acids 1 - 1216, or with the amino acids 1 - 1279 of the SEQ ID NO: 1, wherein the variant polypeptide is 1279 amino acids or less in size.

In an embodiment, the amino acid sequence of the variant polypeptide has at least 80 %, but less than 100 % amino acid sequence identity with amino acids 1-1279 of SEQ ID NO: 1; and at least one substitution, insertion or deletion of an amino acid, the at least one substitution, insertion or deletion preventing N-glycosylation of an amino acid at the amino acid position corresponding to the amino acid position 672 of the of the SEQ ID NO: 1,when expressed in a host cell capable of N-glycosylation.

In an embodiment, the amino acid sequence of the variant polypeptide has at least 80 %, but less than 100 % amino acid sequence identity with amino acids 1-1279 of SEQ ID NO: 1; and at least one amino acid substitution at amino acid position corresponding to the amino acid position selected from 672, 623, 674 of the SEQ ID NO: 1, and combinations thereof. In an embodiment, the amino acid sequence of the variant polypeptide has at least 80 %, but less than 100 % amino acid sequence identity with amino acids 1-1279 of SEQ ID NO: 1; and at least one amino acid deletion at amino acid position corresponding to the amino acid position selected from 672, 623, 674 of the SEQ ID NO: 1, and combinations thereof. In an embodiment, the amino acid sequence of the variant polypeptide has at least 80 %, but less than 100 % amino acid sequence identity with amino acids 1-1279 of SEQ ID NO: 1; and at least one amino acid insertion between amino acids corresponding to the amino acids 672 and 673 of the SEQ ID NO: 1, and/or between the amino acids corresponding to the amino acids 673 and 674 of the SEQ ID NO: 1.!In an embodiment, the variant polypeptide has an increased fructanase productivity and/or improved fructan degrading performance, when compared to a wild type parent polypeptide of the variant polypeptide, or to a polypeptide having the sequence SEQ ID NO: 1.

For purposes of the present invention, fructanase productivity may be determined according to the method described in the Example 2, and the fructan degrading performance (activity) may be determined according to the method described in the Example 3.

In an embodiment, the variant polypeptide has an improved fructan degrading performance, when compared to its wild type parent polypeptide, or to the polypeptide having the sequence SEQ ID NO: 1, meaning, the variant polypeptide has a higher fructanase activity when compared to a wild type parent polypeptide of the variant polypeptide, or to a polypeptide having the sequence SEQ ID NO: 1.

In an embodiment, the relative fructanase activity of the variant polypeptide is at least 2, preferably at least 4 fold higher when compared to a wild type parent polypeptide of the variant polypeptide, or to a polypeptide having the sequence SEQ ID NO: 1. In an embodiment, the relative fructanase activity of the variant polypeptide is at least 10, preferably at least 20, more preferably at least 30, even more preferably at least 40 fold higher, when compared to a wild type parent polypeptide of the variant polypeptide, or to a polypeptide having the sequence SEQ ID NO: 1. In an exemplary embodiment, the variant polypeptide has at least 21 fold, at least 31 fold, at least 32 fold, at least 37 fold, or at least 46 fold higher fructanase activity when compared to a polypeptide having the sequence SEQ ID NO: 1. By fructanase activity fold change, such as increase, in this respect is meant the ratio between the fructanase activity of the measured variant (B) and fructanase activity of the comparison sample (A), e.g. the polypeptide having the sequence SEQ ID NO: 1. Thus for fructanase activities of (A) and (B) the fold change of B with respect to A is B/A.

In an embodiment, the variant polypeptide has an improved fructan degrading performance when compared to the wild type parent polypeptide of the variant polypeptide, or to a polypeptide having the sequence SEQ ID NO:1, at least when produced in a cell capable of N-glycosylation. In an embodiment, the variant polypeptide has an increased fructanase productivity when compared to a wild type parent polypeptide of the variant polypeptide, or to a polypeptide having the sequence SEQ ID NO: 1.

In an embodiment, the relative fructanase productivity of the variant polypeptide is at least 2 fold, preferably at least 3 fold, more preferably at least 4 fold, more preferably at least 6 fold, even more preferably at least 8 fold higher when compared to a polypeptide having the sequence SEQ ID NO: 1. By fructanase productivity fold change, such as increase, in this respect is meant the ratio between the fructanase productivity of the measured variant (B) and fructanase productivity of the comparison sample (A), e.g. the polypeptide having the sequence SEQ ID NO: 1. Thus for fructanase productivities of (A) and (B) the fold change of B with respect to A is B/A.

In an embodiment, the increased fructanase productivity of the variant polypeptide means, the variant polypeptide also has an improved fructan degrading performance, when compared to a polypeptide having the sequence SEQ ID NO: 1 or when compared to a wild type parent polypeptide of the variant polypeptide, as the quantity of the variant polypeptide is increased.

In an embodiment, the fructanase productivity of the variant polypeptide is higher when compared to a wild type parent polypeptide of the variant polypeptide, or to a polypeptide having the sequence SEQ ID NO: 1, wherein the higher fructanase productivity of the variant polypeptide is independent of the host cell wherein the variant polypeptide is produced. In an embodiment, the variant polypeptide has an improved fructan degrading performance and/or improved fructanase activity when compared to a wild type parent fructanase of the variant polypeptide, or to a polypeptide having the sequence SEQ ID NO:1, at least when produced in a fungal cell.

In an embodiment, the variant polypeptide having the at least one substitution, insertion or deletion of an amino acid preventing N-glycosylation of an amino acid at the amino acid position corresponding to the amino acid position 672 of the of the SEQ ID NO: 1, has a higher fructanase productivity and/or activity, when compared to a polypeptide having the sequence SEQ ID NO: 1.

In an embodiment, the variant polypeptide having the at least one substitution, insertion or deletion of an amino acid preventing N-glycosylation of an amino acid at the amino acid position corresponding to the amino acid position 672 of the of the SEQ ID NO: 1, has a higher fructanase productivity and/or activity, when compared to its wild type parent fructanase polypeptide. In an embodiment, the variant polypeptide having the at least one substitution, insertion or deletion of an amino acid preventing N-glycosylation of an amino acid at the amino acid position corresponding to the amino acid position 672 of the of the SEQ ID NO: 1, has a higher fructanase productivity and/or activity, when compared to a corresponding variant polypeptide having an asparagine (N) at the amino acid position corresponding to the amino acid position 672 of the of the SEQ ID NO: 1 N-glycosylated.

In an embodiment, the variant polypeptide having the at least one substitution, insertion or deletion of an amino acid preventing N-glycosylation of an amino acid) at the amino acid position corresponding to the amino acid position 672 of the of the SEQ ID NO: 1; and being obtained via a C-terminal deletion of SLAP domain amino acids corresponding to the amino acids 1166-1276 of the of the SEQ ID NO: 1, has both, an increased fructanase productivity, and an increased fructanase activity, when compared to a wild type parent polypeptide of the variant polypeptide or to a fructanase polypeptide having the sequence SEQ ID NO: 1.

In an embodiment, the relative fructanase activity and/or productivity of the variant polypeptide is independent of the exact *Lactobacillus* strain of origin of the variant polypeptide. In an embodiment, the variant polypeptide is a variant of *Lactobacillus* fructanase. In an embodiment, the variant polypeptide is a variant of *Lactobacillus crispatus* or *Lactobacillus amylovorus* fructanase.

In an embodiment, the fructan degrading performance and/or relative fructanase productivity of the variant polypeptide is increased when compared to its wild type parent fructanase polypeptide comprising amino acids corresponding to the amino acids of sequence SEQ ID NO: 1. In an embodiment, the relative fructanase activity and/or productivity of the variant polypeptide is increased when compared to its wild type parent fructanase polypeptide having at least 80 % amino acid sequence identity with the amino acids 383 - 858 of SEQ ID NO: 1.

In an embodiment, the variant polypeptide has a substrate specificity to levan over inulin. In an embodiment, the variant polypeptide has a substrate specificity ratio of inulin:levan of 0.8 or less, preferably 0.5 or less, more preferably 0.4 or less or 0.3 or less. In an embodiment, the variant polypeptide has a pH optimum of 4 - 7, preferably of 5 - 6. In an embodiment, the variant polypeptide has a temperature optimum of 40 - 70 °C, preferably of 50 - 70 °C, such as 60 °C. In an embodiment, the variant polypeptide has: a substrate specificity ratio of inulin:levan of 0.8 or less, preferably 0.5 or less; and/or a pH optimum of 5-6; and/or a temperature optimum of 50 - 70 °C. The above-mentioned properties show that the variant polypeptide is stable and active over a wide temperature and pH range. In an embodiment, the variant polypeptide which has fructanase activity and which retains activity in temperatures above ambient temperature is advantageous for applications wherein fructan degradation is required in such process conditions. High temperature tolerance of fructanase has the benefit of increased specific activity.

In an embodiment, the variant polypeptide has the amino acids corresponding to the amino acids 383 - 858 of SEQ ID NO: 1, and a total number of amino acid substitutions and/or insertions and/or deletions in the variant polypeptide compared to SEQ ID NO: 5 is 1 - 10, preferably 1 - 3. In an embodiment, the variant polypeptide has the amino acids corresponding to the amino acids of SEQ ID NO: 5, and a total number of amino acid substitutions and/or insertions in the variant polypeptide compared to SEQ ID NO: 5, is 1 - 10, preferably 1 - 3. In an embodiment, the variant polypeptide is the variant polypeptide of SEQ ID NO: 5.

In an embodiment, the variant polypeptide has at least 80 %, at least 84 %, at least 86 %, at least 88 %, at least 90 %, at least 92 %, at least 94 %, at least 96 %, or at least 98 % amino acid sequence identity with the amino acids of SEQ ID NO: 5. In an embodiment, the variant polypeptide has fructanase activity and consists of amino acids corresponding to the amino acids of SEQ ID NO:5, wherein the variant polypeptide has at least 80 %, at least 84 %, at least 86 %, at least 88 %, at least 90 %, at least 92 %, at least 94 %, at least 96 %, or at least 98 % amino acid sequence identity with the amino acids of SEQ ID NO: 5.

In an embodiment, the variant polypeptide has the amino acid sequence of SEQ ID NO: 2, SEQ ID NO: 4, SEQ ID NO: 5, SEQ ID NO: 6, SEQ ID NO: 7, SEQ ID NO: 8, SEQ ID NO: 11 or SEQ ID NO: 13. In an embodiment, the variant polypeptide consists of the amino acid sequence of SEQ ID NO: 2, SEQ ID NO: 4, SEQ ID NO: 5, SEQ ID NO: 6, SEQ ID NO: 7, SEQ ID NO: 8, SEQ ID NO: 11 or SEQ ID NO: 13.

In an embodiment the variant polypeptide has a predicted molecular weight between 80 - 150 kDa, preferably between 120 - 150 kDa, comprising the amino acids constituting the mature region of the variant polypeptide and without including a signal sequence. In an embodiment the variant polypeptide has a predicted molecular weight of approximately 135 - 145 kDa, wherein the variant comprises all the domains of its parent polypeptide or is without the SLAP domains of its parent polypeptide. In an embodiment the variant polypeptide has a predicted molecular weight of approximately 80 - 90 kDa, wherein the variant is without the C-terminal SLAP, LamG, and Big2 domains of its parent polypeptide. The predicted molecular weight can be determined by calculating the sum of the molecular weights of the individual amino acids in the variant polypeptide.

The present variant of fructanase is advantageous in having good stability and fructanase activity. The variant has different glycosylation pattern compared to a wild type fructanase, which may provide improved specific activity, stability and yield when produced in a host cell. In some embodiments, the present variant of fructanase provides improved yield in production and better performance in use. Thus, in some embodiments the present variant of fructanase is advantageous in having an increased activity. The present variant of fructanase is advantageous in having improved productivity. The present variant of fructanase is especially advantageous in having an improved performance in degrading fructan in the process conditions that require increased activity, such as process conditions with variable temperature and pH. As shown in the examples, the claimed variant polypeptide of fructanase has an activity and level of productivity, which are improved when compared to the wild type parent fructanase.

In an embodiment, a fusion protein comprises the variant polypeptide and at least one: an amino acid sequence providing a secretory signal sequence; an amino acid sequence which facilitates purification, such as an affinity tag or His-tag; an amino acid sequence controlling and/or enhancing the production of the fusion protein, such as a carrier polypeptide; an amino acid sequence encoding a protease cleavage site; a linker sequence; an amino acid sequence having an enzyme activity; and/or an amino acid sequence providing for the fusion protein with binding affinity, such as a carbohydrate binding moiety.

In an embodiment, the fusion protein comprises the amino acid sequence of the variant polypeptide and the at least one amino acid sequence providing a secretory signal sequence, said secretory signal sequence directing the fusion protein to secretory pathway of the host cell wherein the fusion protein is produced. In an embodiment, the fusion protein comprises a signal sequence which is advantageous in the translocation of the variant polypeptide within and out of the host cell. In an embodiment, the signal sequence is with or without a carrier polypeptide sequence.

In an embodiment, the fusion protein comprises an amino acid sequence which facilitates purification, such as His-tag or polyhistidine tag, wherein in the tag comprises a string of histidine residues. In an embodiment, the fusion protein comprises one or more sequences that control and direct the production of the variant polypeptide of fructanase.

In an embodiment, the fusion protein comprises a carbohydrate binding moiety (CBM) as a carrier polypeptide, which is optionally a fragment of another protein or enzyme derived from the same or different organism as the variant polypeptide. The signal sequence and carrier polypeptide may be derived from the same host, or alternatively derived from different hosts. In an embodiment, the fusion protein comprises a signal sequence originating from the same host cell wherein the fusion protein comprising the fructanase variant polypeptide is produced in. In an embodiment the signal sequence originates from *Trichoderma reesei,* wherein the fusion protein comprising the fructanase variant polypeptide is produced in. In another embodiment, the signal sequence originates from another organism than the host cell wherein the fusion protein comprising the fructanase variant polypeptide is produced in.

In an embodiment, the fusion protein comprises an amino acid sequence encoding a protease cleavage site. In an embodiment, the fusion protein comprises an amino acid linker sequence, linking two domains or parts of the fusion protein together.

In an embodiment, the fusion protein comprises an amino acid sequence having an enzyme activity, the said sequence with enzyme activity not being the same sequence as the amino acid sequence encoding the variant polypeptide with fructanase activity.

In an embodiment, the fusion protein comprises a carbohydrate binding moiety amino acid sequence, which provides the fusion protein with binding affinity. In an embodiment, the fusion protein comprises a stabilizer.

In an embodiment an enzyme composition comprises the variant polypeptide or the fusion protein.

In an embodiment, an enzyme composition comprises at least the present variant polypeptide, and optionally a preservative, and/or a carrier. In an embodiment is provided the enzyme composition comprising the variant polypeptide or the fusion protein, and optionally one or more other polypeptides with enzymatic activity. In an embodiment, in addition to the variant polypeptide or the fusion protein, the enzyme composition may also comprise one or more enzymes having hydrolase activity.

In an embodiment the enzyme composition comprising the variant polypeptide of fructanase, or the fusion protein, and one or more additional enzymes, is advantageous in providing a synergistic effect. Such an additional enzyme is desired when the present enzyme composition comprising variant polypeptide, or the fusion protein, is used in e.g., preparation of food products. Therefore, in an embodiment, the present fructanase enzyme is blended with other enzymes thereby obtaining the enzyme composition, to be used in baking, food, feed, and/or technical applications, for example.

Particularly advantageous synergistic enzymes that work with fructanase in food products are inulinases, levanases, endo-fructanases and invertases. Further particularly advantageous synergistic enzymes that work with fructanase in food applications, such as baking applications, are glucoamylases. In an embodiment, the enzyme composition comprising the variant polypeptide of fructanase or the fusion protein comprises an additional enzyme such as an enzyme liberating glucose or maltose. In an embodiment, the enzyme composition comprising the variant polypeptide of fructanase or the fusion protein comprises one or more enzymes selected from the group consisting of amylase, maltogenic amylase, beta amylase, aminopeptidase, carboxypeptidase, catalase, cellulolytic enzyme, chitinase, cutinase, cyclodextrin glycosyltransferase, deoxyribonuclease, esterase, glucan 1,4- alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, xylanase, glucose isomerase, mannitol dehydrogenase and fructose dehydrogenase.

In an embodiment, the enzyme composition comprising the variant polypeptide of fructanase, or the fusion protein, comprises at least one additional enzyme, such as an enzyme used in baking, animal feed, hygiene product or in food, one or more other fructan degrading enzymes, sugar isomerases, sugar transferases and/or sugar dehydrogenases

In an embodiment, the enzyme composition is a liquid composition comprising diluents such as water or buffer, and/or stabilizers, such as sorbitol, glycerol or propylene glycol, and/or preservatives, such as sodium benzoate, potassium sorbate or parabens.

In an embodiment, the enzyme composition is provided in the form of a liquid composition or a solid composition, or mixtures thereof. In an embodiment, the enzyme composition is provided in the form of a solution, dispersion, paste, flour, powder, granule, granulate, coated granulate, tablet, cake, crystal, crystal slurry, gel, or pellet.

The present variant polypeptide comprised by the present fusion protein and/or the present enzyme composition is advantageous, as it has an increased fructanase activity and/or fructanase productivity, when compared, for example, to a polypeptide according to SEQ ID NO: 1. The present variant polypeptide, the present fusion protein and the present enzyme composition are advantageous, for example, for the degradation of fructan in grain materials, cereals, vegetables, and fruits.

The present variant polypeptide is preferably recombinantly produced non-naturally occurring protein. It can be prepared using generally known recombinant DNA technology. Briefly, the polynucleotide encoding a variant polypeptide is cloned and inserted into an expression vector, transformed into a host cell, and then expressed. Preferably, mutations are introduced into the coding sequence with codons preferred by the selected host strain. Methods for protein production by recombinant technology in different host systems are known in the art. Preferably, the variants are produced as extracellular proteins that are secreted into the culture medium wherein the host cell is cultured, and from which they can be easily recovered and isolated.

In an embodiment, a recombinant host cell comprises genetic elements that allow producing at least one variant polypeptide or the fusion protein.

In an embodiment, the recombinant host cell is a microorganism, selected from the group consisting of: eukaryotic fungal cells or prokaryotic bacterial cells, preferably the host cell is selected from filamentous fungal cells such as *Trichoderma* or *Aspergillus,* or from gram-positive Bacilli such as *Bacillus,* most preferably the host cell is *Trichoderma reesei.*

In an embodiment, the recombinant host cell is a fungal cell, preferably a filamentous fungal cell such as *Trichoderma, Aspergillus, Thermothelomyces, Myceliophthora, Humicola,* or *Fusarium.* In an embodiment, the recombinant host cell is a filamentous fungal cell such as *Trichoderma reesei, Aspergillus oryzae, Aspergillus niger, Thermothelomyces heterothallica, Myceliophthora thermophila, Humicola insolens,* or *Fusarium venenatum.*

In an embodiment, the recombinant host cell is selected from the group consisting of gram-positive Bacilli such as *Bacillus,* in particular *Bacillus subtilis, Bacillus pumilus, Bacillus alkalophilus, Bacillus amyloliquefaciens, Bacillus brevis, Bacillus circulans, Bacillus clausii, Bacillus coagulans, Bacillus halodurans, Bacillus lautus, Bacillus lentus, Bacillus licheniformis, Bacillus megaterium, Bacillus stearothermophilus,* or *Bacillus thuringiensis.*

In an embodiment, the host cell is selected from yeast cells such as *Saccharomyces cerevisiae, Schizosaccharomyces pombe, Pichia pastoris, Yarrowia lipolytica.* In an embodiment, the host cell is selected from gram-negative bacterial cells, such as a strain of *E. coli.*

In an embodiment, the recombinant host cell belongs to the order *Hypocreales.* In an embodiment, the recombinant host cell belongs to the family *Hypocreaceae.* In an embodiment, the recombinant host cell belongs to the genus *Trichoderma.* In a preferable embodiment, the host cell is *Trichoderma reesei* or *Bacillus subtilis,* more preferably the host cell is *Trichoderma reesei.*

In an embodiment, the recombinant host cell has an increased fructanase productivity when compared to a host cell comprising genetic elements that allow producing a polypeptide having the sequence SEQ ID NO: 1, or when compared to a host cell comprising genetic elements that allow producing the wild type parent polypeptide of the variant polypeptide.

In an embodiment, the recombinant host cell comprises genetic elements that allow producing the at least one variant polypeptide or the fusion protein comprising the amino acid sequence of the variant polypeptide, wherein the recombinant host cell has an increased fructanase secretion when compared to a host cell comprising genetic elements that allow producing a polypeptide or a fusion protein comprising the sequence SEQ ID NO: 1, or when compared to a host cell comprising genetic elements that allow producing the wild type parent polypeptide of the variant polypeptide.

In an embodiment, the relative fructanase productivity of the recombinant host cell comprising the genetic elements that allow producing the at least one variant polypeptide, is higher when compared to a host cell comprising genetic elements that allow producing fructanase according to sequence SEQ ID NO: 1, or when compared to a host cell comprising genetic elements that allow producing the wild type parent polypeptide of the variant polypeptide, wherein the higher fructanase productivity of the recombinant host cell is not dependent of the host cell wherein the variant polypeptide is produced.

In an embodiment, the relative fructanase productivity of the recombinant host cell is at least 2 fold higher when compared to a host cell comprising genetic elements that allow producing a polypeptide according to SEQ ID NO: 1, or when compared to a host cell comprising genetic elements that allow producing the wild type parent polypeptide of the variant polypeptide. In an embodiment, the relative fructanase productivity of the variant polypeptide is at least 2.5, preferably at least 3, more preferably at least 4, even more preferably at least 6 fold higher, when compared to a to a host cell comprising genetic elements that allow producing a polypeptide according to SEQ ID NO: 1, or when compared to a host cell comprising genetic elements that allow producing the wild type parent polypeptide of the variant polypeptide. In a preferable embodiment, the relative fructanase productivity of the variant polypeptide is at least 10, preferably at least 50, more preferably at least 100 fold higher, when compared to a to a host cell comprising genetic elements that allow producing a polypeptide according to SEQ ID NO: 1, or when compared to a host cell comprising genetic elements that allow producing the wild type parent polypeptide of the variant polypeptide. In an embodiment, the relative fructanase productivity of the recombinant host cell refers to the quantity of the fructanase variant polypeptide produced into the production medium.

The recombinant host cell can be used to produce the variant polypeptide of fructanase and to contain a polynucleotide encoding it. The selection of a specific recombinant host cell can be made in preparation of variants of fructanase with different properties. For example, a host cell can be selected, which provides post-translational modifications beneficial for stability or activity, or which facilitates processing of the variant polypeptide of fructanase produced in the host cell. The host cell can be operably linked to one or more control sequences to direct the production of the variant, and that make it possible to initiate the production of the present variant polypeptide by a stimulus, as is known in the field. In an embodiment the host cell is non-pathogenic. This is particularly advantageous when using the host cell, or the fructanase variant produced in it, for foodstuff, or feedstuff applications.

The present recombinant host cell is advantageous, as it has an increased productivity of the present variant polypeptide and/or the present fusion protein, when compared to, for example, a host cell comprising the wild type parent polypeptide of the variant polypeptide or the polypeptide according to SEQ ID NO: 1. The present recombinant host cell is advantageous, as it produces the present variant polypeptide and/or the present fusion protein having fructanase activity.

In an embodiment, a method of manufacturing the variant polypeptide or the fusion protein comprises: cultivating the recombinant host cell in conditions allowing production of the at least one variant polypeptide or the fusion protein; and optionally recovering the variant polypeptide or the fusion protein.

In an embodiment of the method, the recombinant host cells are separated from the medium comprising the variant polypeptide and/or the fusion protein, an optionally, said medium is concentrated by removing water. The concentrate can be made, for example, by ultrafiltration, microfiltration, evaporation or any other suitable means. In an embodiment, the variant polypeptide and/or the fusion protein is separated from other proteins, optionally this is done by selective crystallization, precipitation, chromatographic methods, or any other suitable means.

The present method of manufacturing the variant polypeptide or the fusion protein is advantageous, as it allows production of the variant polypeptide or the fusion protein having increased fructanase productivity and/or improved fructan degrading performance, when compared to, for example, the wild type parent polypeptide of the variant polypeptide or the polypeptide having the sequence SEQ ID NO: 1.

In an embodiment, the variant polypeptide and/or the fusion protein and/or the enzyme composition is used for degradation of fructan in plant-based material. In an embodiment, the use of the variant polypeptide and/or the fusion protein and/or the enzyme composition refers to a method of using.

In an embodiment, the use comprises bringing the variant polypeptide and/or the fusion protein and/or the enzyme composition in contact with fructan comprised by the plant-based material, thereby allowing the degradation of fructan comprised by said plant-based material into at least fructose. In an embodiment, the use comprises bringing the variant polypeptide and/or the fusion protein and/or the enzyme composition in contact with fructan comprised by the plant-based material, at reaction conditions allowing the degradation of fructan by the variant polypeptide. In an embodiment, plant-based material is fructan containing material.

In an embodiment, the fructan containing materials comprise:

cereals, such as wheat, spelt, rye and barley;
vegetables such as onions, shallots, garlic, leeks, artichoke, asparagus, beets, brussels sprouts, savoy cabbage, fennel, chicory, agave and snow peas;
nuts, such as cashews and pistachios;
pulses, such as kidney beans, black beans, mung means, navy beans, lima beans and split peas; and
fruits such as watermelon, nectarine, grapefruit, persimmon, pomegranate, plums, ripe bananas, dates, prunes and raisins.

In an embodiment, the variant polypeptide and/or the fusion protein and/or the enzyme composition can be used for the degradation of fructan in fructan containing materials. In an embodiment, the variant polypeptide and/or the fusion protein and/or the enzyme composition can be used for the degradation of fructan in grain and cereal containing materials, such as in non-baked products including doughs and premixes for baking, and/or in baked products including breads and cakes. In an embodiment, the variant polypeptide and/or the fusion protein and/or the enzyme composition can be used for the degradation of fructan in vegetable and/or fruit containing foods, such as vegetable containing foodstuff preparations.

In an embodiment, the fructan containing materials comprise processed food and/or convenience food.

In an embodiment, the use of the variant polypeptide and/or the fusion protein and/or the enzyme composition reduces the fructan content of the fructan containing material at least 20 %, preferably at least 30 %, more preferably at least 40 %, even more preferably at least 50 %, most preferably at least 60 %, when compared to the fructan containing material without any fructanase enzyme. In a preferred embodiment, the variant polypeptide and/or the fusion protein and/or the enzyme composition reduces the fructan content of the fructan containing material at least 49 % or 54 %, when compared to the fructan containing material without any fructanase enzyme.

In an embodiment, the variant polypeptide and/or the fusion protein and/or the enzyme composition is used to liberate fructose from fructan. In an embodiment, the use of the variant polypeptide and/or the fusion protein and/or the enzyme composition increases the fructose and glucose content of the fructan containing material, when compared to the fructan containing material without any fructanase enzyme. In an embodiment, the variant polypeptide and/or the fusion protein and/or the enzyme composition reduces the fructan content and increases the fructose content of the fructan containing material when compared to the fructan containing material without any fructanase enzyme.

In an embodiment, the use of the variant polypeptide and/or the fusion protein and/or the enzyme composition comprises a use of a combination of the present variant fructanase and a glucoamylase for degradation of fructan in plant-based material, for keeping the ratio of fructose / glucose constant in the enzyme composition treated plant-based material. In an embodiment, the variant polypeptide and/or the fusion protein and/or the enzyme composition, is brought in contact with plant-based material together with glucoamylase, thereby degrading fructan, and keeping the ratio of fructose / glucose constant in the plant-based material. In such an embodiment, the use, or the method wherein the variant polypeptide and a glucoamylase are used in combination, is a use in baking application, for example.

In an embodiment, the variant polypeptide and/or the fusion protein and/or the enzyme composition is used to degrade fructans in plant-based material, to obtain foodstuff suitable for a diet low in fermentable carbohydrates, known as FODMAP. In an embodiment, the use of the variant polypeptide and/or the fusion protein and/or the enzyme composition is especially beneficial in preparing products comprising plant-based materials suitable for diet low in FODMAP ("Fermentable, Oligo-, Di-, Monosaccharides, and Polyols"), the materials thereby having low fructan content.

In an embodiment, the variant polypeptide and/or the fusion protein and/or the enzyme composition is used to degrade fructans in plant-based material, to obtain fructose sirup, which fructose sirup can be used for further applications, such as alcohol fermentation. In such an embodiment, the use comprises adding the present variant polypeptide and/or the fusion protein and/or the enzyme composition to the application in question, and eventually recovering fructose sirup.

In an embodiment, the use or method of using comprises the use of the variant polypeptide and/or the fusion protein and/or the enzyme composition in baking, and/or in manufacturing feedstuff, foodstuff, a feed additive, a dietary supplement, a pharmaceutical, a detergent, a cleaning product, a hygiene product (such as mouth wash) processed food, and/or animal feed (such as horse feed). In such embodiment, the use comprises adding the present variant polypeptide and/or the fusion protein and/or the enzyme composition to the application in question, such as baking, and mixing.

In an embodiment, the variant polypeptide and/or the fusion protein and/or the enzyme composition is added to the plant-based material, such as flour or dough, in an amount of 0.5-500 mg (corresponding approximately to 10 - 10000 fructanase units, FRU) of the variant polypeptide per kg of the plant-based material. In an exemplary embodiment, the variant polypeptide is added in an amount of 5-100 mg of the variant polypeptide per kg of flour, more preferably in an amount of 20-70 mg of the variant polypeptide per kg of flour.

In an embodiment, the variant polypeptide and/or the fusion protein and/or the enzyme composition is used in preparation artificial sweeteners, such as low-calorie sweeteners (LCS). In an embodiment, the variant polypeptide and/or the fusion protein and/or the enzyme composition is used in preparation of fructose, which is beneficial at least because of low production costs. In an embodiment, the variant polypeptide and/or the fusion protein and/or the enzyme composition is used in manipulating fructan metabolism in plants.

Using the present variant polypeptide and/or the present fusion protein and/or the present enzyme composition is advantageous, as the variant polypeptide is capable of increasing degradation of fructan.

In an embodiment, there is provided a premix for baking, comprising the variant polypeptide and/or the fusion protein and/or the enzyme composition. In an embodiment, the premix for baking comprises at least the variant polypeptide and/or the fusion protein and/or the enzyme composition, and one or more ingredients needed or suitable for baking. In an embodiment, the ingredients needed or suitable for baking include ingredients selected from one or more of flour, cereal grains or flakes, sugar, yeast or other leavening agents, fat or oil, salt, spices, flavorings, bakery ingredients, water, milk or milk alternative and eggs. In an embodiment, the ingredients needed or suitable for baking also include an improver for baking, comprising one or more ingredients from the group consisting of enzymes, wheat gluten, carriers (wheat gluten maltodextrin etc.), emulsifiers, and mono and diglycerides.

The present variant polypeptide, the present fusion protein and the present enzyme composition are advantageous in the premix for baking, as the variant polypeptide is capable of increasing degradation of fructan in the premix and/or in the baking dough wherein the premix is mixed into. This is especially beneficial in preparing baked products suitable for LOW-FODMAP diet, having low fructan content.

In an embodiment, there is provided a method of reducing fructan content in a baked product, the method comprising:

- providing ingredients for baking and mixing said ingredients together with the variant polypeptide and/or the fusion protein and/or the enzyme composition, thereby providing a dough, followed by proofing the dough, the fructan content in the dough being reduced while mixing and proofing; and
- baking the dough thereby providing the baked product having reduced fructan content.

In an embodiment, the method of reducing fructan content in a baked product comprises mixing the variant polypeptide and/or the fusion protein and/or the enzyme to the baking ingredients in an amount of 0.5-500 mg (corresponding approximately to 10 - 10000 fructanase units, FRU) of the variant polypeptide per kg of the plant-based material, such as flour, comprised by the baking ingredients. In an exemplary embodiment, the variant polypeptide is added in an amount of 5-100 mg of the variant polypeptide per kg of flour, more preferably in an amount of 20-70 mg of the variant polypeptide per kg of flour.

The dough produced with said method also has a reduced fructan content, the method thus providing also a method for reducing fructan content in a non-baked product such as dough.

In an embodiment, there is provided a food or foodstuff, comprising the variant polypeptide and/or the fusion protein and/or the enzyme composition. In an embodiment, the food or foodstuff, comprising the variant polypeptide and/or the fusion protein and/or the enzyme composition, is a mixture food product and/or processed food product, such as manufactured plant-based product/processed plant-based product, a supplement, an additive or mixtures thereof.

In an embodiment, there is provided a baked product having reduced fructan content, the baked product comprising the variant polypeptide and/or the fusion protein and/or the enzyme composition, and wherein the fructan content is reduced compared to a baked product without said variant polypeptide and/or the fusion protein and/or the enzyme composition. In an embodiment, fructan content of the baked product is reduced at least 20 %, at least 30 %, at least 40 %, or at least 50 % when compared to a baked product which does not comprise said variant polypeptide and/or the fusion protein and/or the enzyme composition. In a preferable embodiment, fructan content of the baked product is reduced at least 49 %, at least 54 %, when compared to a baked product which does not comprise said variant polypeptide and/or the fusion protein and/or the enzyme composition.

In an embodiment, the baked product comprises the variant polypeptide and/or the fusion protein and/or the enzyme to the baking ingredients in an amount of 0.5-500 mg of the variant polypeptide per kg of the plant-based material, such as flour, comprised by the baked product. In an exemplary embodiment, the baked product comprises 5-100 mg of the variant polypeptide per kg of flour, more preferably in an amount of 20-70 mg of the variant polypeptide per kg of flour.

In an embodiment, there is provided an animal feed or feedstuff, comprising the variant polypeptide and/or the fusion protein and/or the enzyme composition. In an embodiment, the animal feed or feedstuff, comprising the variant polypeptide and/or the fusion protein and/or the enzyme composition, is a dry forage, roughage, pasture, silage, energy feed, protein supplement, mineral supplement, vitamin supplement, additive, or mixtures thereof. In an embodiment the feed is animal feed intended to be fed to animals, such as any compound feed or mixture. In another embodiment feed comprises grains such as maize, wheat, oats, barley, sorghum and rice; protein sources such as soybean meal, sunflower meal and canola meal; and/or one or more minerals.

In an embodiment, there is provided a detergent comprising the variant polypeptide and/or the fusion protein and/or the enzyme composition. In an embodiment, the detergent is provided in the form of a solution, dispersion, paste, powder, granule, granulate, coated granulate, tablet, cake, crystal, crystal slurry, gel, or pellet.

## EXAMPLES

### Example 1. Fructanase variant design

A set of variant polypeptides were designed based on the mature wildtype *Lactobacillus crispatus* fructanase LFRU disclosed in WO2017220864. The variants were designed to have increased fructanase activity and/or productivity when produced in *Trichoderma reesei* host cell. The resulting variant polypeptides comprise a GH32 catalytic domain, and optionally multiple additional putative domains as presented in Figure 1.

The variant polypeptides were designed based on the mature wildtype *Lactobacillus crispatus* fructanase consisting of 1279 amino acids (SEQ ID NO:1). Variant polypeptides with three types of modifications were designed: variants with amino acid substitution preventing N-glycosylation of the asparagine at amino acid position 672, variants with a C-terminal truncation, and variants with combination of both modifications. In some variants designed, two C-terminal SLAP domains were removed by deleting the terminal 114 amino acids, ending at serine in position 1165 (numbering starting from the mature protein sequence presented in SEQ ID NO: 1). In some other variants, a larger C-terminal truncation comprising Big2, LamG, and the two SLAP domains was created by deleting 420 terminal amino acids, ending at threonine in position 859. Four different single amino acid substitutions (N672Q, T674A, T674G and T674V) which all prevent N-glycosylation in the position 672 of the variant polypeptide, were designed and introduced into either the full-length or a truncated version of the variant polypeptide of fructanase. The SEQ ID numbers of the variant polypeptides designed are listed in Table 1.

**Table 1.** List of fructanase variants produced in *T. reesei* and their sequence identifiers. Engineered amino acid sites, listed under modifications, are presented with amino acid numbering corresponding to the mature wildtype (wt) fructanase protein sequence (SEQ ID NO: 1).

| Molecule name | Modification | SEQ ID NO |
|---|---|---|
| LFRU | none, is wt | 1 |
| LFG | T674A | 2 |
| LFS | STOP1166 | 3 |
| LF2 | N672Q, STOP1166 | 4 |
| LF3 | T674A, STOP1166 | 5 |
| LF4 | T674G, STOP1166 | 6 |
| LFS | T674V, STOP1166 | 7 |
| LF3GS | T674A, STOP860 | 8 |

### Example 2. Production of LFRU fructanase and variants thereof in *T. reesei*

Standard molecular biology methods were used in the isolation and enzyme treatments of DNA (e.g., isolation of plasmid DNA, digestion of DNA to produce DNA fragments), in *E. coli* transformations, sequencing etc. The basic laboratory methods used were either as described by the enzyme, reagent or kit manufacturer or as described in the standard molecular biology handbooks, e.g., in (Sambrook & Russell, 2001) or as described in the following examples.

Expression plasmids were constructed for production of wildtype fructanase LFRU and fructanase variants. The genes of interest (GOIs) encoding the designed fructanase variant (Table 1) were ordered as synthetic genes using codons optimized for expression in *T. reesei.* The fructanases were expressed from *T. reesei* cbh1 (cel7A) promoter using an N-terminal carrier polypeptide (CBM and linker) encoding sequence from *T. reesei* cbh2 (cel6A). A Kex2 protease cleavage site was included between the carrier polypeptide and the fructanase as described in (Paloheimo et al., 2003). Transcription was terminated using *T.reesei* cbh2 (cel6A) terminator, and a synthetic amdS marker gene was used for selecting transformants. In addition, the fructanase expression constructions contain cbh1 (cel7A) 5' and 3' flanking regions for optional targeting of the expression vector into the cbh1 (cel7A) locus. A schematic picture of the expression plasmids is presented in Figure 2.

Circular expression plasmids were transformed in *T. reesei* protoplasts. The transformants were selected on plates containing acetamide as the sole nitrogen source. The *T. reesei* host strain used is lacking four main endogenous cellulases: CBHI/Cel7A, CBHII/Cel6A, EGI/Cel7B and EGII/Cel5A. The transformations were performed according to (Penttilä et al., 1987), with the modifications described in (Karhunen et al., 1993). Alternatively, CR!SPR-CAS technology can be used in transformations.

Transformants were purified on selection plates through single conidia prior to sporulating them on potato dextrose agar (PDA). The fructanase production of the transformants was analyzed from the culture supernatants of shake flask cultivations (50 mL). Transformants were grown for 7 days at 30 °C, 250 rpm in a complex cellulase-inducing medium (Joutsjoki et al., 1993) buffered with 5 % (w/v) $KH_2PO_4$ at pH 6.0.

Production of the recombinant proteins was detected from the culture supernatants by SDS-polyacrylamide gel electrophoresis. The supernatant samples included production supernatants from transformants producing the fructanase variants of Table 1. A distinct high-molecular weight band (-120 kDa) was detected in the samples of all the fructanase supernatants analyzed, except for the variant LF3GS, which contains a large C-terminal truncation leading to a protein band of about 80 kDa in SDS-PAGE (Figure 3). Based on the SDS-PAGE analysis, the variant fructanases having prevented N-glycosylation of the amino acid at position 672, and optionally comprising a C-terminal truncation, have increased fructanase production (lanes 4 - 10 in Fig. 3), when compared to the wildtype LFRU fructanase (lane 3 in Fig. 3). Hence, deglycosylation of the amino acid/ asparagine at position 672 and/or removal of the C-terminal domains comprising at least the SLAP domains from the wildtype fructanase of SEQ ID NO: 1, resulted in fructanase variants having increased fructanase productivity. Most of the variants comprising the combined modification of C-terminal truncation and blocked N-glycosylation at position corresponding to the amino acid position 672 of the SEQ ID NO: 1, also have higher fructanase productivity when compared to the variant LFG (only comprises the substitution T674A) or to the variant LFS (only C-terminal truncation) (Fig. 3). This indicates, said combined modification in variants has the further effect of increasing fructanase productivity to a yet higher level, which cannot be observed with variants comprising either of these two modifications (C-terminal truncation or blocked N-glycosylation) alone.

## Example 3. Analysis of fructanase activity of LFRU variants produced in *T. reesei*

The enzyme activity of the recombinant fructanase variants was measured from the culture supernatant as the release of reducing sugars (i.e., fructose residues) from inulin using Fluent® automation workstation (Tecan Group Ltd, Männedorf, Switzerland).

The samples were diluted in a 0.05 M sodium acetate buffer pH 5.0, and the substrate, inulin from chicory (Sigma No. I2255), was dissolved in the same buffer. A substrate concentration of 0.25 % (w/v) was used. Sample dilution (25 µL) was mixed with the preheated substrate (225 µL) in the DeepWell plate and incubated at 37 °C, for 15 min. After 15 min incubation, 375 µL dinitrosalicylic reagent (10 g of 3,5-dinitrosalicylic acid, 16 g of NaOH, 300 g of potassium sodium tartrate tetrahydrate dissolved in distilled water and filled-in to 1 liter) was added, and the reaction was stopped by boiling the mixture for 8 min. Samples were cooled for 5 min at 4 °C and thereafter for 3 min at 20 °C. After the cooling step 200 µL of each reaction mixture was transferred into a 96 well microplate. The absorbance was measured at 540 nm in a microplate spectrophotometer (Tecan, Spark).

A calibration curve was obtained using fructose as the standard (7.5, 10, 14, 20, 30, 40 and 50 µmol/mL). Fructose standards were treated identically to the samples. One enzymatic unit was defined as an amount of the enzyme which produces 1 µmol of reducing sugars per mL. The amount of released fructose is calculated from the calibration curve. A relative fructanase activity of the measured samples was calculated as fold change in reference to the wildtype enzyme LFRU (SEQ ID NO:1).

The results showing the relative fructanase activity of the fructanase variants are shown in Table 2. The relative fructanase activity measured from the culture supernatant of the wildtype fructanase (LFRU; SEQ ID NO: 1) and of the truncated variant with the SLAP-domain deleted (LFS; SEQ ID NO: 3) was at the level of the host background, i.e., no fructanase activity was detected for these fructanases when produced in *T. reesei* in the current setup. An increased relative fructanase enzyme activity was recovered for the full-length fructanase variant (LFG; SEQ ID NO: 2) with an amino acid substitution T674A. The results indicate that blocking or prevention of glycosylation of the asparagine at position 672 leads to production of a fructanase with increased activity thus having improved fructan degrading performance. A combination of a C-terminal truncation with the selected mutation at the position 672 (LF2) or 674 (LF3-LF5 and LF3GS) resulted in variants having even further increased fructanase activity, as indicated in the Table 2. Hence, the results indicate the C-terminal truncation alone in the fructanase variants mainly increases fructanase productivity, whereas a mutation preventing the N-glycosylation of an amino acid at position corresponding to the amino acid position 672 of SEQ ID NO:1 increases fructanase productivity (see Fig. 3) as well as improves fructan degrading performance (see Table 2). Further, preventing the N-glycosylation of the amino acid at position corresponding to the amino acid position 672 of SEQ ID NO:1 seems to be required to produce an active enzyme in *T. reesei.* The variant polypeptides comprising the combined modification of C-terminal truncation and blocked N-glycosylation at position 672, have clearly increased productivity and also higher fructanase activity, when compared to the wild type fructanase LFRU and to the truncated fructanase LFS. Most of the variants comprising said combined modification also have higher fructanase activity when compared to the variant LFG which only comprises the amino acid substitution T674A. This indicates, said combined modification in variants has the further effect of increasing fructanase activity to a yet higher level, which cannot be observed with variants comprising either of these two modifications (C-terminal truncation or blocked N-glycosylation) alone.

**Table 2.** The relative activity of the *Lactobacillus crispatus* LFRU fructanase variants compared to the wildtype enzyme produced in *T. reesei.* The activity was measured from the shake flask culture supernatants. All samples were analyzed in duplicates.

| Fructanase molecule | Modification | SEQ ID NO | Relative fructanase activity |
|---|---|---|---|
| None | Empty host | none | 1 |
| LFRU | wt | 1 | 1 |
| LFG | T674A | 2 | 21 |
| LFS | STOP1166 | 3 | 2 |
| LF2 | N672Q, STOP1166 | 4 | 21 |
| LF3 | T674A, STOP1166 | 5 | 37 |
| LF4 | T674G, STOP1166 | 6 | 31 |
| LF5 | T674V, STOP1166 | 7 | 32 |
| LF3GS | T674A, STOP860 | 8 | 46 |

**Example 4. Characterization of LFRU fructanase variant LF3 produced in *T. reesei***

The pH optimum of the recombinant fructanase variant LF3 was determined in the universal McIlvaine's buffer within a pH range of 4.0 - 8.0, using inulin as a substrate. The reaction was carried out at 40 °C for 10 min. The LF3 fructanase is active in relatively broad pH range (4 - 6.5), the optimum being at pH 5 - 6 (see Fig. 6 (A)). Temperature dependency of the fructanase activity was determined at pH 5 (10 min). The optimal temperature for enzymatic activity of LF3 was determined to be 60 °C (see Fig. 6 (B)).

Substrate specificity of the fructanase variant LF3 was determined by high-performance liquid chromatography (HPLC). Two substrates, inulin from chicory (Sigma No. I2255) and Timothy grass levan (P-LEVAN, Magazyme), were used in the analysis. One mL of 2 % substrate solution was equilibrated at 40 °C for 5 min, followed by addition of 20 μL of the sample (diluted in 50 mM acetate buffer at pH 5.0). Reaction mixtures were incubated at 40 °C for 10 min and thereafter enzymatic reaction was terminated by boiling the tubes for 5 min. After boiling, samples were cooled to room temperature and filtered through 0.2 μm membrane before HPLC analysis.

Hydrolytic reaction products (liberated fructose) from the reaction mixtures were analyzed and quantified by HPLC as follows. A Dionex DC5000+ ion chromatography system equipped with the ED40 electrochemical detector and Dionex CarboPac PA1 analytical column was used for the analysis. A sample (25 μL) of each reaction product was applied into the column, followed by elution with two solvents: 0.1 M aqueous sodium hydroxide (A) and 0.1 M aqueous sodium hydroxide containing 0.5 M sodium acetate (B). Elution of the reaction products was started with 0.1 M sodium hydroxide solution for 10 minutes at a flow rate of 0.5 mL/minute. Thereafter, a linear gradient of 0 - 90 % solvent B in 0.1 M sodium hydroxide was applied for 30 min. Detected fructose peak area was calculated using Chromelon 7.3 software (Thermo Fisher Scientific Inc.) and used for determination of inulinase / levanase activity ratio. The peak area detected with inulin substrate was about 45 % of the peak area obtained with levan substrate (see Fig. 6 (C)). The result indicates that LFRU fructanase and variants thereof are more active against levan than inulin.

**Example 5. Baking trials of LFRU fructanase variants LF2 and LF3 produced in *T. reesei***

*T. reesei* strains producing either LF2 or LF3 fructanase variant were cultivated in fermentors in complex cellulase inducing medium to obtain material for baking application tests. Culture filtrates were concentrated, and spray dried for baking trials. The LF2 and LF3 fructanase variants were tested for their ability to decrease fructan content of the baked products.

The test bread recipe included 1961 g wheat flour mix, 33.5 g salt, 80 g fresh yeast, 55 g rapeseed oil, 1575 g tap water and 2.36 g of the respective fructanase variant enzyme composition (LF2 or LF3), wherein the 2.36 g fructanase variant enzyme composition contains approximately 100 mg fructanase enzyme variant. Bread without any fructanase enzyme was used as a reference sample. The dough making, proofing and baking conditions are described in the Table 3.

**Table 3.** Dough making, proofing and baking conditions used in the trials.

| **Dough making** | **Conditions** |
|---|---|
| Mixing | 2 + 7 min |
| Temperature | 25 °C |
| Rest time | 15 min |
| Piece weight | 400 g |
| **Proofing** | **Conditions** |
| Temperature | 37 °C |
| Humidity | 67 % |
| Time | 60 - 65 min |
| **Baking** | **Conditions** |
| Temperature | 215 - 225 °C |

Fructan content of the baked breads was determined in duplicates using Megazyme K-FRUC method. The fructan content of the breads baked with LF2 fructanase enzyme was decreased by 49 % (0.22 g fructan/ 100 g bread) and with LF3 fructanase enzyme was decreased by 54 % (0.20 g fructan / 100 g bread), compared to the reference bread baked without the enzyme (0.44 g fructan / 100 g bread). These results indicate that the variant fructanases used in the baking trials efficiently reduce the fructan content of baked products, when mixed in the dough together with other ingredients. Thus, the variant fructanases can be used in degradation of fructan in plant-based material, such as a dough comprising flour.

**Example 6. Production of fructanases from other *Lactobacillus* strains in *T. reesei***

Other *Lactobacillus* fructanases were searched from databases and the synthetic genes were used to produce fructanase recombinant enzymes in *T. reesei* (Table 4). Variants were designed based on the mature wildtype fructanase of *L. amylovorus* LFM24 (SEQ ID NO: 9) and *L. crispatus* LFM33 (SEQ ID NO: 12). The C-terminal SLAP domain deletion and N-glycosylation mutation were designed comparably to *L. crispatus* LFRU fructanase (SEQ ID NO: 1) variants LFG and LFS. The position for amino acid substitution resulting in prevention of N-glycosylation of an amino acid of the variant polypeptide at amino acid position corresponding to blocked N-glycosylation at position 672 of *L. crispatus* LFRU, was determined based on sequence alignment as shown in Figure 4.

**Table 4.** List of fructanases from other *Lactobacillus* strains and their sequence identifiers. Engineered amino acid sites, listed under modifications, are presented with amino acid numbering corresponding to the respective mature wildtype fructanase protein sequence (i.e., LFM24 or LFM33).

| **Source** | **Database ID** | **Identity to SEQ ID NO: 1** | **Molecule designation** | **Modification** | **SEQ ID** |
|---|---|---|---|---|---|
| *Lactobacillus* | R5ZF23 | 93% | LFM24 | wt | 9 |
| *Lactobacillus* | none | 85% | LFM24S | STOP1146 | 10 |
| *Lactobacillus* | none | 85% | LFM24GS | T655A, STOP1146 | 11 |
| *Lactobacillus* | A0A2M9WP68 | 99% | LFM33 | wt | 12 |

(continued)

| Source | Database ID | Identity to SEQ ID NO: 1 | Molecule designation | Modification | SEQ ID |
|---|---|---|---|---|---|
| *Lactobacillus* | none | 90% | LFM33GS | T674A, STOP1166 | 13 |

Expression plasmids were designed for *L. amylovorus* wildtype molecule LFM24 (SEQ ID NO: 9), a C-terminal truncation variant LFM24S (SEQ ID NO: 10), and a variant LFM24GS (SEQ ID NO: 11) containing both the C-terminal truncation and an N-glycosylation mutation (amino acid substitution) T655A. In addition, a variant (LFM33GS; SEQ ID NO: 13) with combined C-terminal truncation and N-glycosylation mutation was designed for a second *L. crispatus* fructanase sequence (SEQ ID NO: 12). *T.reesei* strains for the above-described molecules were constructed and shake flask cultivations performed as described in Example 2.

Production of the recombinant fructanases was detected from the culture supernatant of LFM24, LFM24GS, and LFM33GS by SDS-polyacrylamide gel electrophoresis. Distinct high-molecular weight bands were detected in the samples of all the tested variants LFM24GS, and LFM33GS, while the wildtype *L. amylovorus* LFM24 (SEQ ID NO: 9) was produced in minute amounts in *Trichoderma* (Figure 5, lane 3). Fructanase enzyme activity of the produced heterologous fructanases was determined as described in Example 3. Empty host strain and *L. crispatus* LFRU fructanases were used as references. The results (Table 5) show that the *L. amylovorus* variant LFM24S with the C-terminal truncation is not active, while the LFM24GS containing both C-terminal truncation and the N-glycosylation mutation are clearly active against inulin substrate. Also, the corresponding *L. crispatus* fructanase variant LFM33GS is active. These results indicate that preventing N-glycosylation of an amino acid of the variant polypeptide at amino acid position corresponding to the amino acid position 672 of SEQ ID NO:1 enables production of an enzymatically active fructanase in *Trichoderma.* These results also further indicate that variant LFM24GS comprising the combined modification of C-terminal truncation and blocked N-glycosylation, have increased productivity when compared to the wild type fructanase (LFM24). These results also further indicate that variants LFM24GS and LFM33GS comprising said combined modification also have higher fructanase activity, when compared to the wild type fructanase LFM24 and to the truncated fructanase LFM24S.

**Table 5.** The relative activity of the fructanase variants compared to the wildtype *L. crispatus* LFRU fructanase enzyme produced in *T. reesei.* The activity was measured from the shake flask culture supernatants.

| Fructanase molecule | Modification | SEQ ID | Relative fructanase activity |
|---|---|---|---|
| None | Empty host | none | 1 |
| LFRU | wt | 1 | 1 |
| LFS | STOP1166 | 3 | 2 |
| LFM24 | wt | 9 | 2 |
| LFM24S | STOP1146 | 10 | 4 |
| LFM24GS | T655A, STOP1146 | 11 | 36 |
| LFM33GS | T674A, STOP1166 | 13 | 26 |

**Example 7. Analysis of fructanases from the other *Lactobacillus* strains**

The pH and temperature optima, and the substrate specificity of the recombinant fructanase variants of *L. amylovorus* LFM24GS and *L. crispatus* LFM33GS were determined as described in Example 4. *L. crispatus* LF3 fructanase was used as a reference. As shown in Figure 6, the pH (A) and temperature (B) optima of all molecules are pH 5 - 6 and 60 °C, respectively. Also, all three variant fructanases LF3, LFM24GS and LFM33GS prefer levan as a substrate (Fig. 6 (C)). The results show that the fructanase variants, obtained from wild type parent fructanases isolated from different *Lactobacillus* strains, all comprising comparable modifications (e.g. N-glycosylation of an amino acid of the variant polypeptide at amino acid position corresponding to the amino acid position 672 of SEQ ID NO:1 is prevented), all share comparable characteristics.

Various embodiments have been presented. It should be appreciated that in this document, words comprise, include, and contain are each used as open-ended expressions with no intended exclusivity.

The foregoing description has provided by way of non-limiting examples of particular implementations and embodiments a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed example embodiments may be used to advantage without the corresponding use of other features. As such, the foregoing description shall be considered as merely illustrative of the principles of the present invention, and not in limitation thereof. Hence, the scope of the invention is only restricted by the appended patent claims.

**References**

Joutsjoki VV, Torkkeli TK, and Nevalainen KMH. (1993) Transformation of Trichoderma reesei with the Hormoconis resinae glucoamylase P (gamP) gene: production of a heterologous glucoamylase by Trichoderma reesei. Curr. Genet. 24: 223-228.

Karhunen T, Mäntylä A, Nevalainen KMH and Suominen P. (1993) High frequency one-step gene replacement in Trichoderma reesei. I. Endoglucanase I over production. Mol. Gen. Genet. 241:515 - 522.

Mirdita M, Schütze K, Moriwaki Y, Heo L, Ovchinnikov S, Steinegger M. (2022) ColabFold: Making protein folding accessible to all. Nature Methods 19, 679-682.

Needleman and Wunsch, (1970) A general method applicable to the search for similarities in the amino acid sequence of two proteins. J. Mol. Biol. 48: 443-453.

Paloheimo M, Mäntylä A, Kallio J, and Suominen P. (2003) High yield production of a bacterial xylanase in the filamentous fungus Trichoderma reesei requires a carrier polypeptide with an intact domain structure. Appl. Env. Microbiol. 69: 7073-7082.

Penttilä M., Nevalainen H., Rättö M, Salminen E and Knowles J. 1987. A versatile transformation system for the cellulolytic filamentous fungus Trichoderma reesei. Gene 61:155-164.

Rice P, Longden I, Bleasby A. (2000) EMBOSS: The European Molecular Biology Open Software Suite, Trends Genet. 16: 276-277.

Sambrook J, and Russell DW. (2001) Molecular cloning, a laboratory manual. Cold Spring Harbor Laboratory, New York, US.


**Claims**

**1.** A variant polypeptide having:

fructanase activity;
an amino acid sequence having at least 80 % amino acid sequence identity with the amino acids 383 - 858 of SEQ ID NO: 1; and
at least one substitution, insertion or deletion of an amino acid, the at least one substitution, insertion or deletion preventing N-glycosylation of an amino acid of the variant polypeptide at amino acid position corresponding to the amino acid position 672 of SEQ ID NO: 1, when expressed in a host cell capable of N-glycosylation.

**2.** The variant polypeptide of claim 1, wherein the at least one amino acid substitution or deletion is at amino acid position corresponding to amino acid position 672, 673, or 674 of SEQ ID NO: 1, or any combination thereof.

**3.** The variant polypeptide of claim 1 or 2, wherein the at least one insertion of an amino acid is between the amino acids corresponding to the amino acids 672 and 673 of the SEQ ID NO: 1, or between the amino acids corresponding to the amino acids 673 and 674 of the SEQ ID NO: 1.

**4.** The variant polypeptide of any preceding claim, wherein

- the at least one amino acid substitution or deletion is at amino acid position N672, G673, T674 or any combination thereof; and/or
- the at least one amino acid insertion is between amino acid positions N672 and G673, or between amino acid positions G673 and T674;

wherein the amino acid numbering of the amino acid positions corresponds to the amino acid numbering of SEQ ID NO: 1.

**5.** The variant polypeptide of any preceding claim, wherein the at least one amino acid substitution is selected from N672Q, G673P, T674A, T674G, and T674V, or combinations thereof, wherein the amino acid numbering of the amino acid substitutions corresponds to the amino acid numbering of SEQ ID NO: 1.

**6.** The variant polypeptide of any preceding claim, wherein the size of the variant polypeptide is 476 - 1279 amino acids, 476 - 1223 amino acids, 476 - 1165 amino acids, 476 - 964 amino acids, 476 - 867 amino acids, 476 - 858 amino acids, 476 - 590 amino acids, or 476 - 482 amino acids, preferably the variant polypeptide is 476 - 1165 amino acids in size.

**7.** The variant polypeptide of any preceding claim, having amino acids corresponding to amino acids 1 - 1279 of the SEQ ID NO: 1 or less, amino acids 1 - 1223 or less, amino acids 1 - 1165 or less, amino acids 1 - 964 or less, amino acids 1 - 867 or less, amino acids 1 - 858 or less, amino acids 269 - 858 or less, or amino acids 377 - 858 or less.

**8.** The variant polypeptide of any preceding claim, having at least 85 %, preferably at least 90 %, more preferably at least 95 %, even more preferably at least 98 % amino acid sequence identity with the amino acids 383 - 858 of SEQ ID NO: 1.

**9.** The variant polypeptide of any preceding claim, having an increased fructanase productivity and/or improved fructan degrading performance, when compared to a wild type parent polypeptide of the variant polypeptide, or to a polypeptide having the sequence SEQ ID NO: 1.

**10.** A fusion protein, comprising the variant polypeptide according to any one of claims 1-9, and at least one:

an amino acid sequence providing a secretory signal sequence;
an amino acid sequence which facilitates purification, such as an affinity tag or His-tag;
an amino acid sequence controlling and enhancing the production of the fusion protein, such as a carrier polypeptide;
an amino acid sequence encoding a protease cleavage site;
a linker sequence;
an amino acid sequence having an enzyme activity; and/or
an amino acid sequence providing for the fusion protein with binding affinity, such as a carbohydrate binding moiety.

**11.** An enzyme composition comprising the variant polypeptide of any one of claims 1-9 or the fusion protein of claim 10.

**12.** A recombinant host cell comprising genetic elements that allow producing at least one variant polypeptide of any one of claims 1-9, or the fusion protein of claim 10.

**13.** A method of manufacturing the variant polypeptide of any one of claims 1-9, or the fusion protein of claim 10, comprising:

cultivating the recombinant host cell of claim 12 in conditions allowing production of the at least one variant polypeptide of any one of claims 1-9, or the fusion protein of claim 10; and
optionally recovering the variant polypeptide or the fusion protein.

**14.** Use of the variant polypeptide of any one of claims 1-9, and/or the fusion protein of claim 10 and/or the enzyme composition of claim 11, for degradation of fructan in plant-based material.

**15.** A premix for baking, comprising the variant polypeptide of any one of claims 1-9, and/or the fusion protein of claim 10 and/or the enzyme composition of claim 11.

Fig. 1

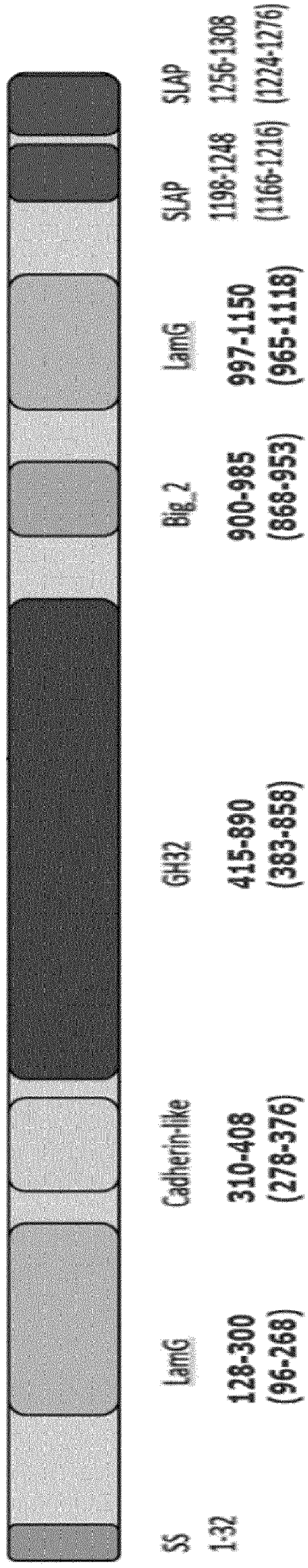
SS
1-32
LamG
128-300
(96-268)
Cadherin-like
310-408
(278-376)
GH32
415-890
(383-858)
Big_2
900-985
(868-953)
LamG
997-1150
(965-1118)
SLAP
1198-1248
(1166-1216)
SLAP
1256-1308
(1224-1276)

Fig. 2

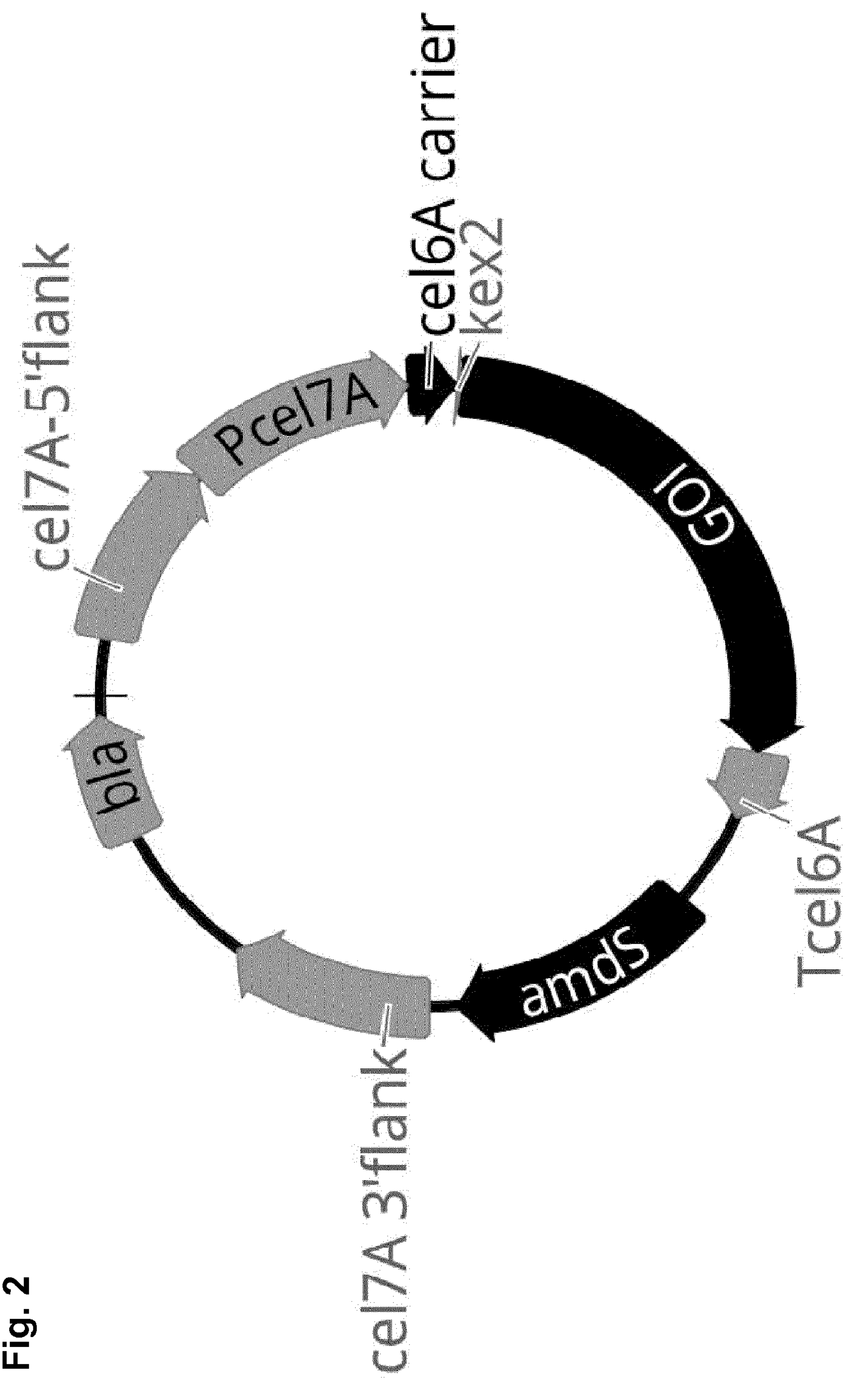
cel6A carrier
kex2
Pcel7A
cel7A-5'flank
GOI
bla
Tcel6A
amdS
cel7A 3'flank

Fig. 3

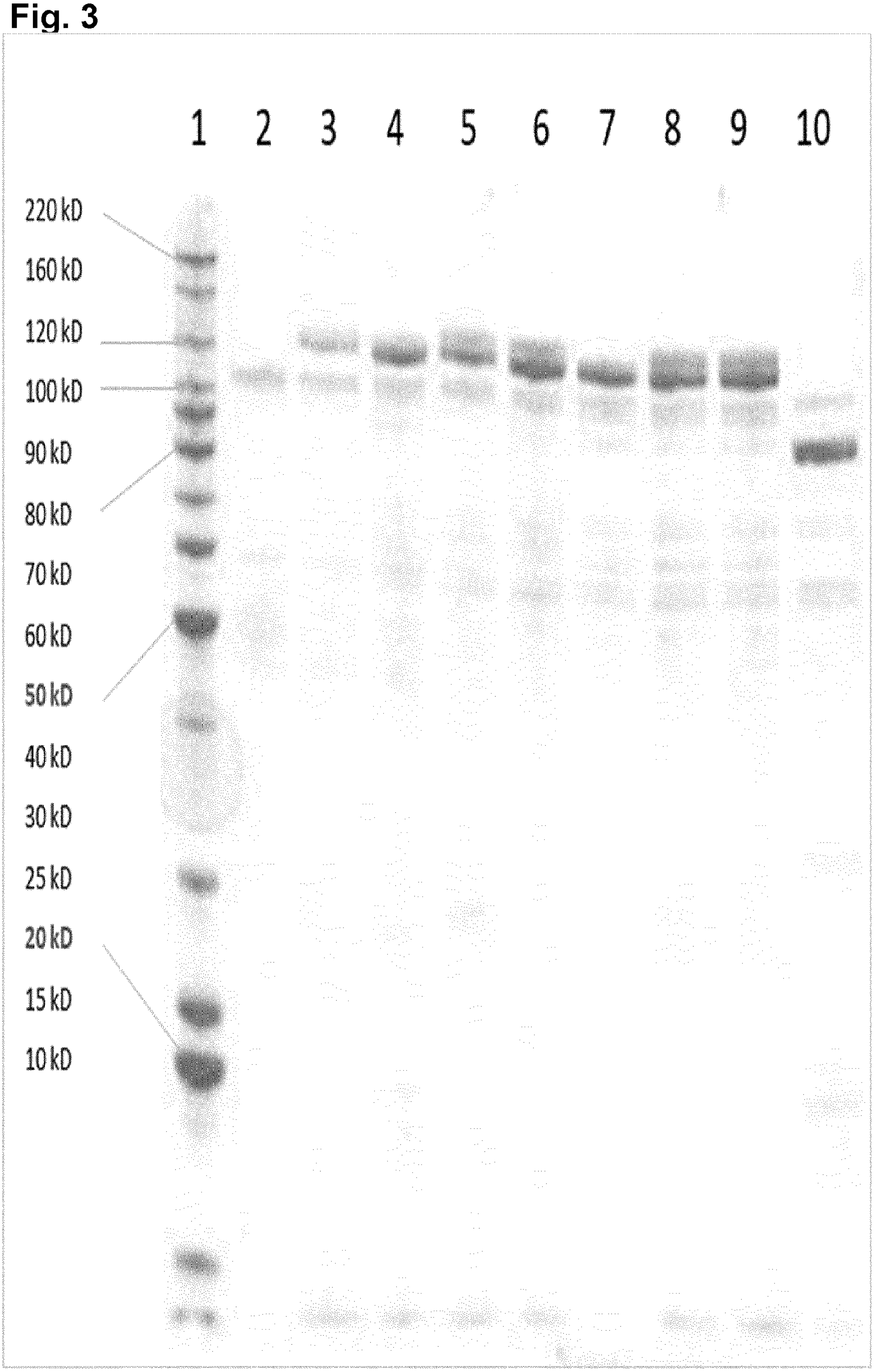
1 2 3 4 5 6 7 8 9 10
220 kD
160 kD
120 kD
100 kD
90 kD
80 kD
70 kD
60 kD
50 kD
40 kD
30 kD
25 kD
20 kD
15 kD
10 kD

Fig. 4

```
SEQ ID NO 1     631 tyyqhsfgtekdptlpdiieenwmnnwdycnlvgntvgqsfngtynlnlkvglvkdgdkylltqtpikay
SEQ ID NO 12    631 tyyqhsfgtekdptlpdiieenwmnnwdycnlvgntvgqsfngtynlnlkvglvkdgdkylltqtpikay
SEQ ID NO 9     612 tyyqhsfgtekdptlpdiieenwmnnwdycnlvgntvgqsfngtynlnlkvglvkdgdkylltqtpikay
```

Fig. 5

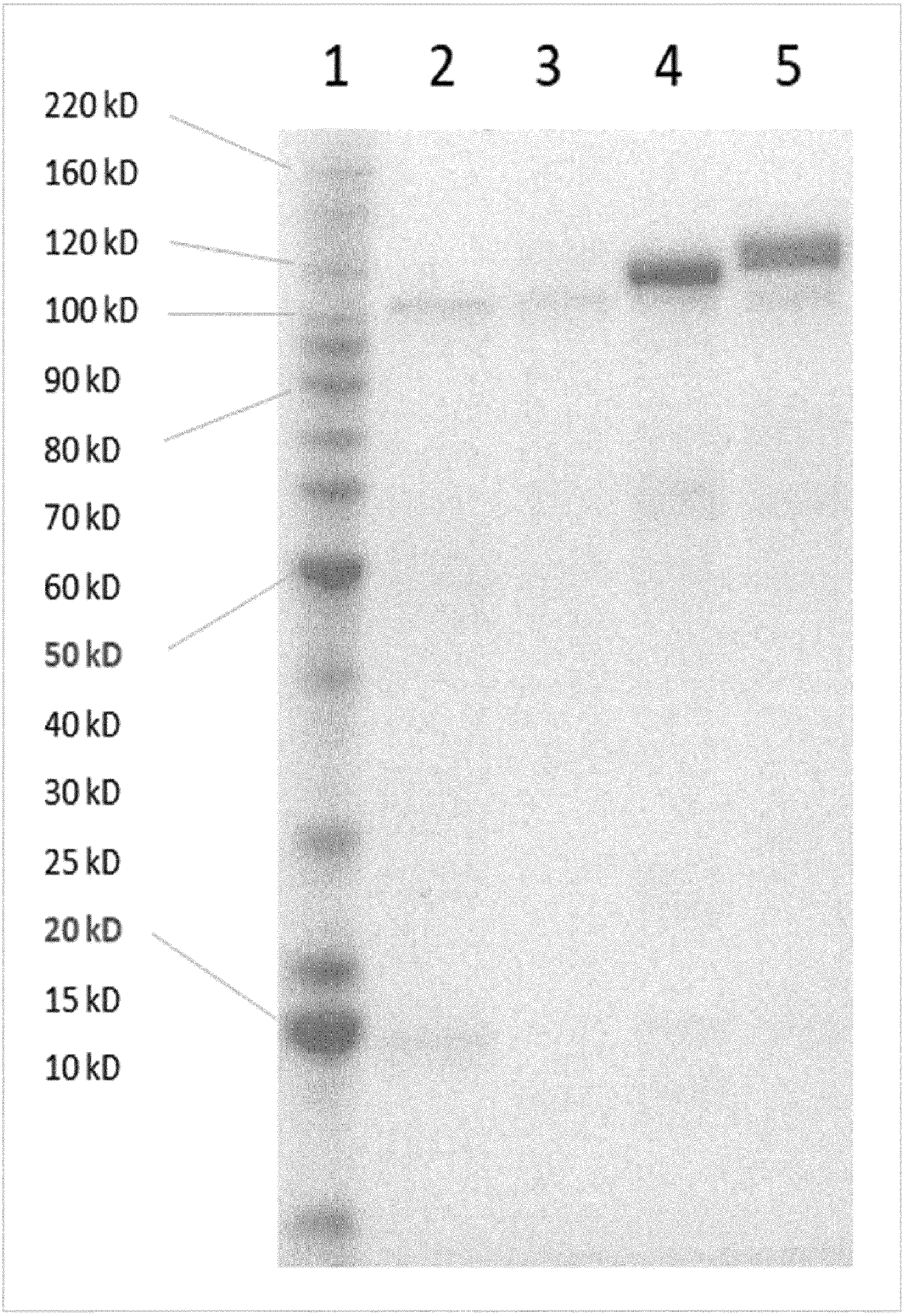
1
2
3
4
5
220 kD
160 kD
120 kD
100 kD
90 kD
80 kD
70 kD
60 kD
50 kD
40 kD
30 kD
25 kD
20 kD
15 kD
10 kD

**Fig. 6**

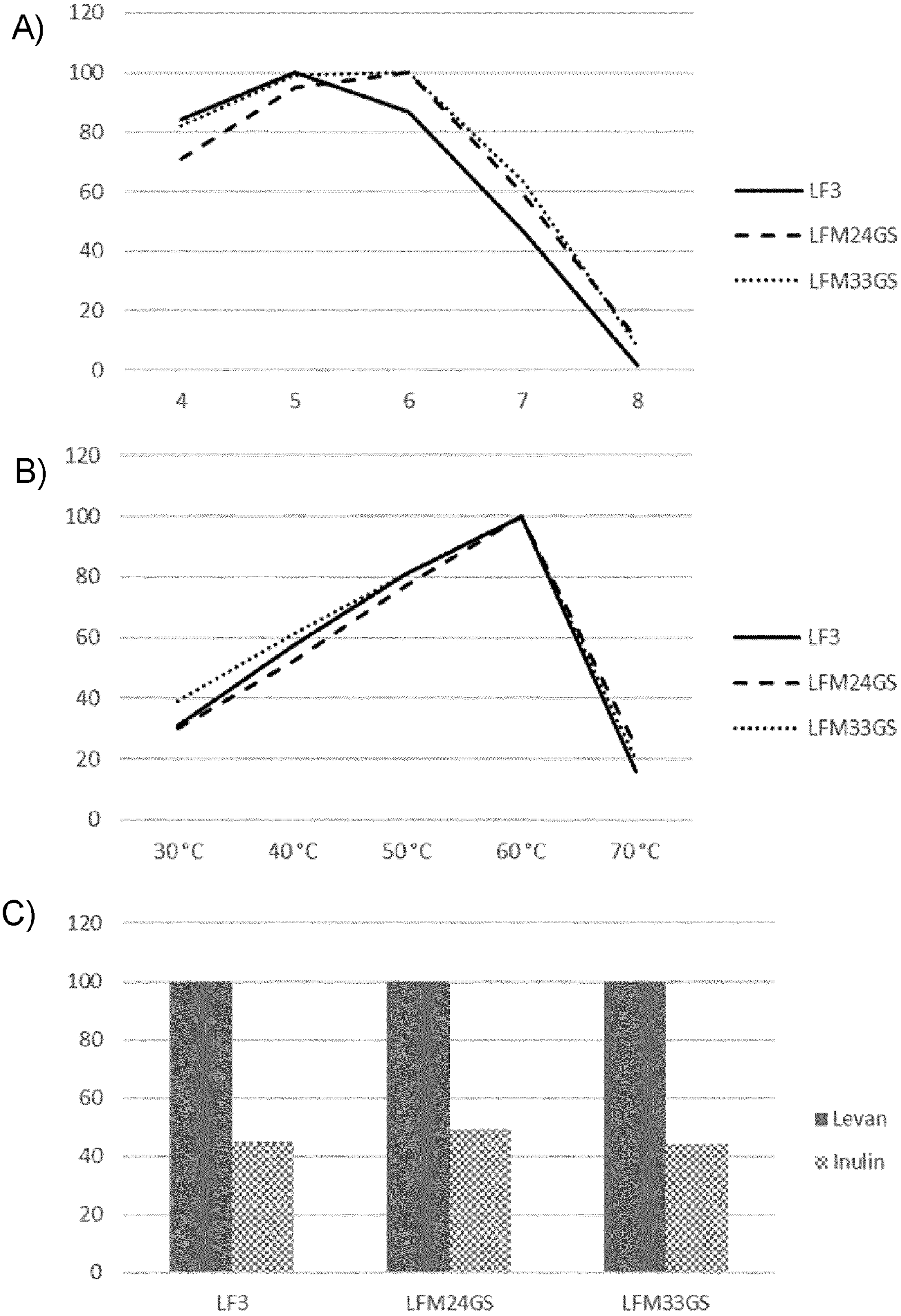
A)
120
100
80
60
40
20
0
4
5
6
7
8
LF3
LFM24GS
LFM33GS
B)
120
100
80
60
40
20
0
30 °C
40 °C
50 °C
60 °C
70 °C
LF3
LFM24GS
LFM33GS
C)
120
100
80
60
40
20
0
LF3
LFM24GS
LFM33GS
Levan
Inulin

Europäisches Patentamt
European Patent Office
Office européen des brevets

# EUROPEAN SEARCH REPORT

Application Number
EP 23 19 2547

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2017/220864 A1 (FAZER AB OY KARL [FI]) 28 December 2017 (2017-12-28)<br>* the whole document *<br>----- | 1-15 | INV.<br>C12N9/38 |
| A | MARTÍNEZ DUNIESKY ET AL: "A thermostable exo-[beta]-fructosidase immobilised through rational de",<br>FOOD CHEMISTRY, ELSEVIER LTD, NL,<br>vol. 145, 28 August 2013 (2013-08-28), pages 826-831, XP028741862,<br>ISSN: 0308-8146, DOI: 10.1016/J.FOODCHEM.2013.08.073<br>* the whole document *<br>----- | 1-15 | |
| A | WO 2022/020665 A2 (KIWI HEALTH INC [US]) 27 January 2022 (2022-01-27)<br>* the whole document *<br>* in particular SEQ ID NO:7 *<br>----- | 1-15 | |
| A | KLOTZ COURTNEY ET AL: "Deletion of S-Layer Associated Ig-Like Domain Protein Disrupts the Lactobacillus acidophilus Cell Surface",<br>FRONTIERS IN MICROBIOLOGY,<br>vol. 11, 17 March 2020 (2020-03-17), XP093109776,<br>Lausanne<br>ISSN: 1664-302X, DOI: 10.3389/fmicb.2020.00345<br>* abstract *<br>----- | 1-15 | TECHNICAL FIELDS SEARCHED (IPC)<br>C12N |

2

The present search report has been drawn up for all claims

| Place of search | Date of completion of the search | Examiner |
|---|---|---|
| Munich | 7 December 2023 | Strobel, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 19 2547

This annex lists the patent family members relating to the patent documents cited in the above-mentioned European search report. The members are as contained in the European Patent Office EDP file on
The European Patent Office is in no way liable for these particulars which are merely given for the purpose of information.

07-12-2023

| Patent document cited in search report | Publication date | Patent family member(s) | Publication date |
|---|---|---|---|
| WO 2017220864 A1 | 28-12-2017 | AU 2017281684 A1 | 06-12-2018 |
| | | CA 3023665 A1 | 28-12-2017 |
| | | DK 3475419 T3 | 18-10-2021 |
| | | EP 3475419 A1 | 01-05-2019 |
| | | ES 2896774 T3 | 25-02-2022 |
| | | FI 127298 B | 15-03-2018 |
| | | NZ 748482 A | 26-08-2022 |
| | | RU 2018136950 A | 23-07-2020 |
| | | US 2019174773 A1 | 13-06-2019 |
| | | US 2020296976 A1 | 24-09-2020 |
| | | WO 2017220864 A1 | 28-12-2017 |
| WO 2022020665 A2 | 27-01-2022 | AU 2021310923 A1 | 09-02-2023 |
| | | CA 3186646 A1 | 27-01-2022 |
| | | EP 4185691 A2 | 31-05-2023 |
| | | US 2023303993 A1 | 28-09-2023 |
| | | WO 2022020665 A2 | 27-01-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017220864 A


### Non-patent literature cited in the description


- **JOUTSJOKI VV** ; **TORKKELI TK** ; **NEVALAINEN KMH**. Transformation of Trichoderma reesei with the Hormoconis resinae glucoamylase P (gamP) gene: production of a heterologous glucoamylase by Trichoderma reesei. *Curr. Genet.*, 1993, vol. 24, 223-228
- **KARHUNEN T** ; **MÄNTYLÄ A** ; **NEVALAINEN KMH** ; **SUOMINEN P**. High frequency one-step gene replacement in Trichoderma reesei. I. Endoglucanase I over production. *Mol. Gen. Genet.*, 1993, vol. 241, 515-522
- **MIRDITA M** ; **SCHÜTZE K** ; **MORIWAKI Y** ; **HEO L** ; **OVCHINNIKOV S** ; **STEINEGGER M**. ColabFold: Making protein folding accessible to all. *Nature Methods*, 2022, vol. 19, 679-682
- **NEEDLEMAN** ; **WUNSCH**. A general method applicable to the search for similarities in the amino acid sequence of two proteins. *J. Mol. Biol.*, 1970, vol. 48, 443-453
- **PALOHEIMO M** ; **MÄNTYLÄ A** ; **KALLIO J** ; **SUOMINEN P.** High yield production of a bacterial xylanase in the filamentous fungus Trichoderma reesei requires a carrier polypeptide with an intact domain structure. *Appl. Env. Microbiol.*, 2003, vol. 69, 7073-7082
- **PENTTILÄ M.** ; **NEVALAINEN H.** ; **RÄTTÖ M** ; **SALMINEN E** ; **KNOWLES J.** A versatile transformation system for the cellulolytic filamentous fungus Trichoderma reesei. *Gene*, 1987, vol. 61, 155-164
- **RICE P** ; **LONGDEN I** ; **BLEASBY A**. EMBOSS: The European Molecular Biology Open Software Suite. *Trends Genet.*, 2000, vol. 16, 276-277
- **SAMBROOK J** ; **RUSSELL DW**. Molecular cloning, a laboratory manual. Cold Spring Harbor Laboratory, 2001